# EUROPEAN PATENT APPLICATION

(11) **EP 4 592 276 A1**
(43) Date of publication of application: **30.07.2025**
(21) Application number: 24154403.0
(22) Date of filing: 29.01.2024
(51) Int. Cl.: C07D 209/16, C07D 405/12, C07C 53/18, C07C 57/15, C07C 309/04, A61P 25/00, A61K 31/4045

(54) **NOVEL ACYLOXYMETHYL PRODRUGS OF PSILOCIN AND RELATED 4-HYDROXYTRYPTAMINES OBTAINED BY AN EFFICIENT SYNTHETIC APPROACH**

(71) Applicant: Universität Heidelberg, 69117 Heidelberg (DE)
(72) Inventor: Klein, Christian D., 69214 Eppelheim (DE); Stirn, Judith L., 69115 Heidelberg (DE)
(74) Representative: Müller-Boré & Partner Patentanwälte PartG mbB

(57) **Abstract**

The present invention relates to a prodrug derived from 4-hydroxytryptamines as well as the use of such prodrug as a medicament and in the treatment of psychiatric disorders.

## Description

The present invention relates to prodrugs derived from 4-hydroxytryptamines as well as their medical uses in general and in the treatment of psychiatric disorders.

Major depressive disorder (MDD) and related psychiatric disorders constitute a considerable disease burden. Since the approval of the first serotonin (5-HT) reuptake inhibitor in 1987, no significantly new treatment modalities have been introduced into clinical practice. A large fraction of depressive individuals does not respond to currently available therapies, and the resulting condition is denoted as treatment-resistant depression (TRD). Furthermore, currently available treatments often have to be administered over extended periods, may be very difficult to withdraw (e.g., Venlafaxine), and/or are associated with patient acceptance and compliance problems (e.g., electroconvulsive therapy). Therefore, a significant clinical need for novel therapeutic interventions for MDD and especially TRD exists.

Recently, renewed attention in the neuropsychiatric field has been directed towards 5-HT_{2A} agonists such as lysergic acid diethylamide (LSD) and psilocin (cf. e.g., Car-hart-Harris et al. J. Psychopharmacol. 2017, 31, 1091). The latter is generally administered as the phosphate ester prodrug psilocybin, which is, in contrast to psilocin, stable against aerobic oxidation. Upon oral administration in humans, psilocybin is absorbed in the small intestine and immediately cleaved by alkaline phosphatases to yield psilocin (cf. Tylš et al. Eur. Neuropsychopharmacol. 2014, 24, 342).

The 5-HT_{2A} receptor agonists psilocin/psilocybin and LSD are frequently referred to as the "classical" or serotonergic psychedelics. In addition to these, compounds with other mechanisms of action, referred to as non-classical psychedelics, are currently being studied in clinical trials and have entered clinical practice. Non-classical psychedelics with therapeutic potential include, in particular, ketamine and MDMA (cf. e.g., Heifets et al. Neuropsychopharmacology 2023, 49, 104).

Both classical and non-classical psychedelics are considered and currently evaluated as therapeutic interventions for conditions beyond depression. Potential applications include post-traumatic stress disorder (PTSD) (cf. e.g., Henner et al. J. Neurol. Sci. 2022, 439, 120302), depressions related to somatic disease and terminal illness (cf. e.g., Ross et al. Neuropharmacology 2022, 216, 109174), neurodegenerative disorders such as Alzheimer's disease (cf. Kozlowska et al. J. Neurochem. 2022, 162, 89), anorexia nervosa (c.f. Majić et al. Nat Med 2023, 29, 1906), and others.

Here, the synthesis and structure-property relationships of 4-hydroxytryptamine prodrugs from two classes are described: ester and acyloxymethyl derivatives (cf. Scheme 1).

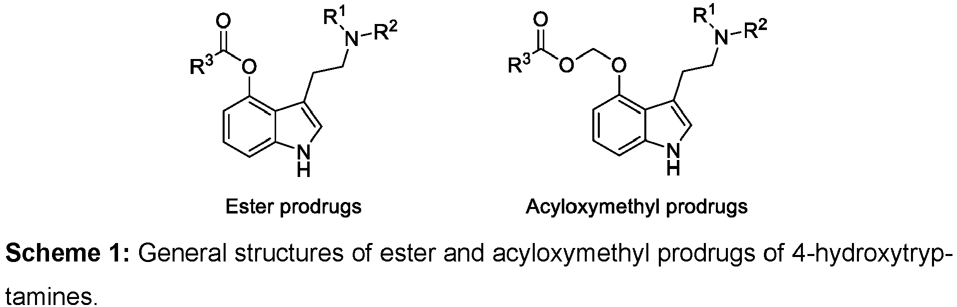

Referring to Scheme 2, besides the natural product psilocybin **2,** synthetic prodrugs of psilocin 1 have been described. This encompasses, in particular, esters of psilocin with various acids. Several carboxylate esters 3 of psilocin have been described by Hofmann et al. (US 3 075 992 A). More recently, carbonate esters 4 (cf. WO 2022/038299 A1), thiocarbonates (cf. e.g., US 11 707 447 B1), and acyloxymethyl prodrugs 5 of psilocin have been reported (cf. WO 2022/038299 A1 and WO 2023/023347 A1). 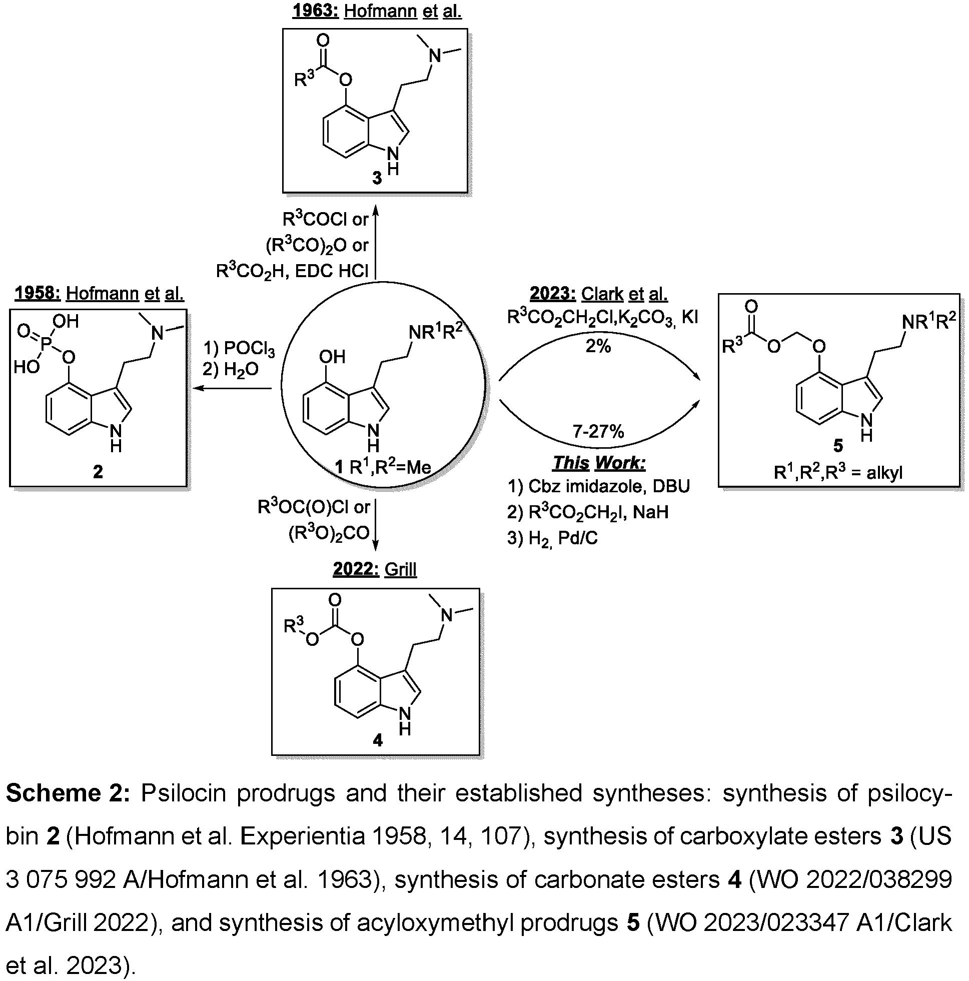 However, a systematic search for analogs with a suitable pharmacokinetic and physicochemical profile for application routes beyond peroral or intravenous administration is still lacking. In particular, such application routes are buccal, sublingual, nasal, inhalatory, and transdermal delivery.

Many of these application routes can ensure a fast onset of the subjective effects during psychedelic treatment settings with lower inter-individual variability than oral psilocybin dosage. This is expected to eliminate undesired waiting times, increase the efficiency of therapist interactions, and improve the predictability of the evoked subjective experience. In contrast, the desired subjective experience may be at risk upon peroral administration, if gastrointestinal absorption is delayed and protracted and, as a consequence, the required plasma concentrations of the drug are not reached. Such an unpredictable pharmacokinetic behavior can occur upon peroral administration, since gastric emptying and gastrointestinal absorption is highly dependent on multiple factors that can only partially be controlled, such as previous food intake and metabolic status of the patient (cf. Vinarov et al. Eur. J. Pharm. Sci. 2021, 162, 105812).

Further, a dosing with fast onset allows the adjustment of the psychedelic experience, if, for example, patient and therapist decide during the session to increase the intensity of the experience (re-dosing), or if it is desired to titrate the dosage depending on the tolerance and desire of the patient. Such a titration dosing may be preferred in cases where the therapist or the patient is uncertain about the tolerance towards the psychedelic experience. In a psychedelic treatment setting, re-dosing and titration cannot reasonably be performed by intravenous or other invasive and potentially threatening forms of drug administration, since this would be incompatible with the intimate and preferentially non-clinical treatment setting. Intravenous administration can, for example, in many countries only be performed in rooms that fulfil certain legal requirements and therefore do not provide the desired sense of sociability. For the settings described above, in which fast dosing and release is desired, a prodrug with short half-life in human blood plasma in the order of minutes is required. In addition, the prodrug should be sufficiently stable in saliva and other secretions so that no degradation occurs before the prodrug has been absorbed. A premature cleavage of the prodrug, i.e. drug release before entering the bloodstream, would result in the presence of a highly potent 5-HT receptor agonist in a high concentration at mucosal tissues. This can lead to disturbances of the local blood circulation, increase of thrombocyte activity, increase of peristaltic activity in the gastrointestinal tract, and other undesired and harmful adverse effects (cf. Mohammad-Zadeh et al. J. Vet. Pharmacol. Ther. 2008; 31, 187). It is therefore undesirable to use the biologically active 5-HT receptor agonist for trans-mucosal application while an inactive prodrug releasing the active drug only after reaching the blood circulatory system is highly preferred for the administration of serotonergic psychedelics.

In light of the current evaluation and promise of psychedelic treatments for diseases in which, for example, the swallowing capacity of patients is restricted or absent, as in Alzheimer's disease, application routes of 4-hydroxytryptamine prodrugs are required that rely neither on the patient's ability to swallow or on injection. Swallowing is frequently restricted in neurodegenerative diseases such as Alzheimer's disease and injections are a relatively drastic procedure, particularly if repeated, i.e., if regular administration is desired. In this setting, a fast drug release in human plasma may not be essential and an extended release can potentially be tolerated better, or even be advantageous due to a less sudden onset of action. These benefits are illustrated by the considerable success of externally applied patches for the transdermal delivery of opioid analgesics (cf. e.g., Kress, Eur. J. Pain 2009, 13, 219).

Going beyond the therapeutic setting into the potential applications of small, repeated or continuous doses of psychedelics (micro-dosing) as neuro-enhancing drugs, the administration of suitable skin-permeating prodrugs formulated in transdermal patches appears highly attractive. For the latter application, in which a constant exposure and extended drug release is required, compounds with a slow cleavage rate in human plasma or tissue may be preferable over prodrugs with a fast release.

Taken together, there is considerable potential for application routes beyond peroral or injectable formulations in the field of serotonergic psychedelic drugs, and the respective formulations should contain inactive prodrugs that are stable until entering the bloodstream.

Accordingly, in view of the prior art, an object underlying the present invention is to provide prodrugs derived from 4-hydroxytryptamines which can be applied in medicine, particularly, in treating psychiatric disorders. It is a further objective of the present invention to provide the use of such prodrugs in medicine and, particularly, in treating psychiatric disorders. An even further object underlying the present invention is to provide a novel synthesis method for producing acyloxymethyl derivatives of hydroxytryptamines having a high and more reliable yield.

The solution to the above technical problems is provided by the embodiments characterized in the claims.

Accordingly, the present invention relates to a prodrug having the formula (1) or salts thereof, 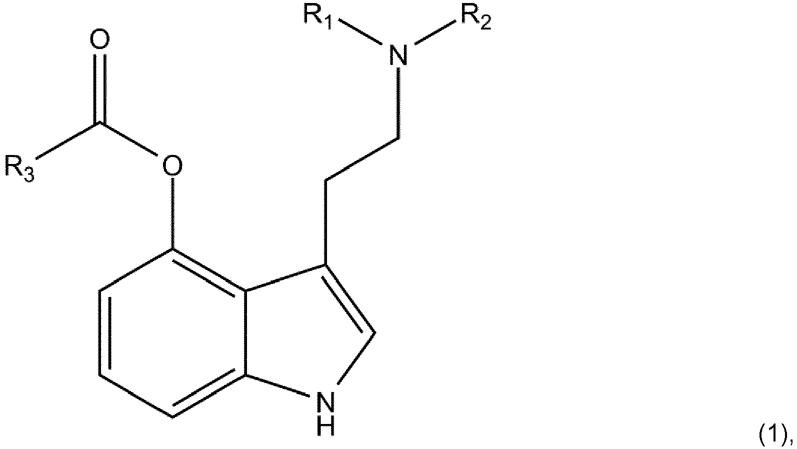
wherein R₃ is selected from the group consisting of cyclopropyl, isopropyl, n-propyl, isobutyl, tert-butyl, neopentyl, phenyl, and 2-furyl residues; and,
provided that R₃ is selected from the group consisting of cyclopropyl, isopropyl, and 2-furyl residues, R₁ and R₂ are selected from the group consisting of methyl, ethyl, n-propyl, isopropyl, n-butyl, tert-butyl residues and combinations thereof;
and, provided that R₃ is selected from the group consisting of n-propyl, isobutyl, *tert-*butyl, neopentyl, and phenyl residues, R₁ and R₂ are not the same and are selected from the group consisting of methyl, ethyl, *n*-propyl, isopropyl, *n*-butyl, and *tert*-butyl residues; and
wherein, in the salts of formula (1), the product of said formula (1) is protonated.

Further, the present invention relates to a prodrug for use as a medicament, the prodrug having the formula (1) or salts thereof, 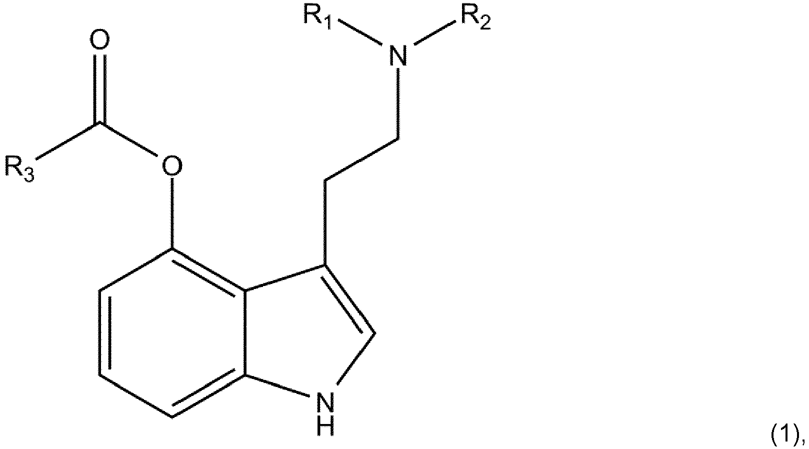
wherein R₃ is selected from the group consisting of cyclopropyl, isopropyl, *n*-propyl, isobutyl, *tert*-butyl, neopentyl, phenyl, and 2-furyl residues; and,
provided that R₃ is selected from the group consisting of cyclopropyl, isopropyl, and 2-furyl residues, R₁ and R₂ are selected from the group consisting of methyl, ethyl, *n-*propyl, isopropyl, *n*-butyl, *tert*-butyl residues and combinations thereof;
and, provided that R₃ is selected from the group consisting of *n*-propyl, isobutyl, *tert-*butyl, neopentyl, and phenyl residues, R₁ and R₂ are not the same and are selected from the group consisting of methyl, ethyl, *n*-propyl, isopropyl, *n*-butyl, and *tert*-butyl residues; and
wherein, in the salts of formula (1), the product of said formula (1) is protonated.

Even further, the present invention relates to a prodrug for use in the treatment of psychiatric disorders, the prodrug having the formula (1) or salts thereof, 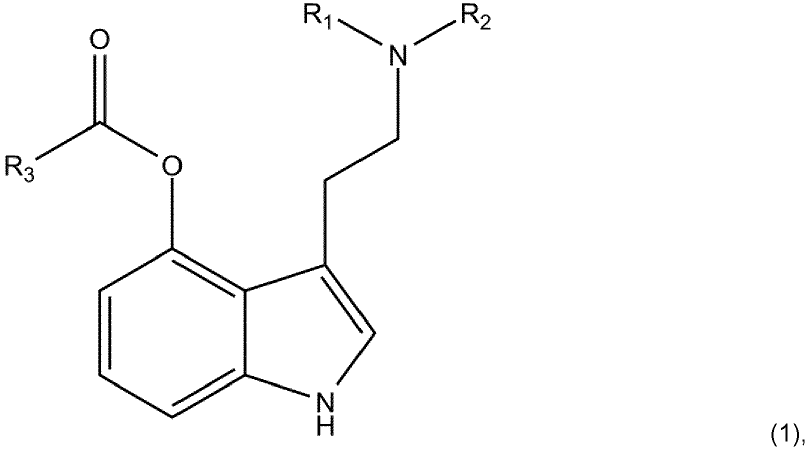
wherein R₃ is selected from the group consisting of cyclopropyl, isopropyl, *n*-propyl, isobutyl, *tert*-butyl, neopentyl, phenyl, and 2-furyl residues; and,
provided that R₃ is selected from the group consisting of cyclopropyl, isopropyl, and 2-furyl residues, R₁ and R₂ are selected from the group consisting of methyl, ethyl, *n-*propyl, isopropyl, *n*-butyl, *tert*-butyl residues and combinations thereof;
and, provided that R₃ is selected from the group consisting of *n*-propyl, isobutyl, *tert-*butyl, neopentyl, and phenyl residues, R₁ and R₂ are not the same and are selected from the group consisting of methyl, ethyl, *n*-propyl, isopropyl, *n*-butyl, and *tert*-butyl residues; and
wherein, in the salts of formula (1), the product of said formula (1) is protonated.

Still further, the present invention relates to a prodrug having the formula (2) or salts thereof, 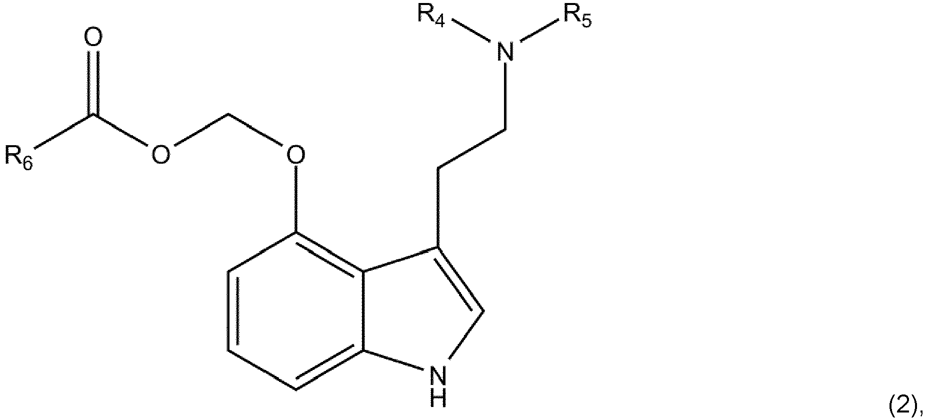
wherein R₆ is selected from the group consisting of cyclopropyl, isopropyl, *n*-propyl, isobutyl, *tert*-butyl, neopentyl, phenyl, and 2-furyl residues; and,
provided that R₆ is selected from the group consisting of isopropyl and 2-furyl residues, R₄ and R₅ are selected from the group consisting of methyl, ethyl, *n*-propyl, isopropyl, *n*-butyl, *tert*-butyl residues and combinations thereof;
and, provided that R₆ is selected from the group consisting of *n*-propyl, cyclopropyl, isobutyl, *tert*-butyl, neopentyl, and phenyl residues, R₄ and R₅ are not the same and are selected from the group consisting of methyl, ethyl, *n*-propyl, isopropyl, *n*-butyl, and *tert*-butyl residues; and
wherein, in the salts of formula (2), the product of said formula (2) is protonated.

Moreover, herein described is a prodrug for use as a medicament, the prodrug having the formula (2) or salts thereof, 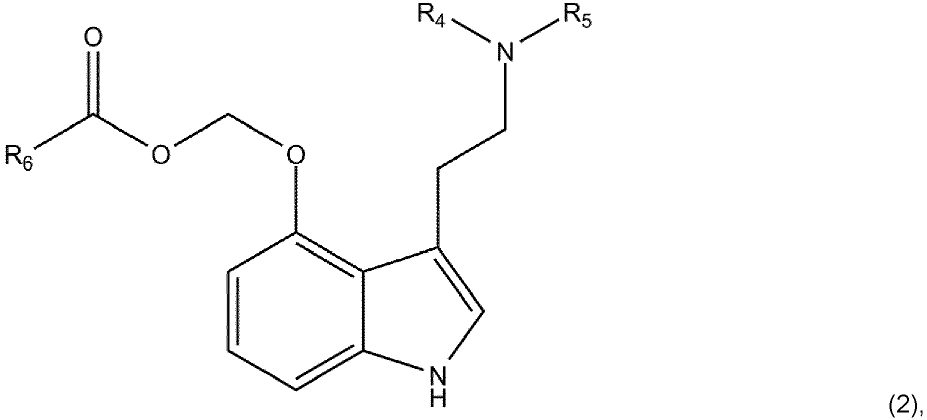
wherein R₆ is selected from the group consisting of cyclopropyl, isopropyl, *n*-propyl, isobutyl, *tert*-butyl, neopentyl, phenyl, and 2-furyl residues; and,
provided that R₆ is selected from the group consisting of isopropyl and 2-furyl residues, R₄ and R₅ are selected from the group consisting of methyl, ethyl, *n*-propyl, isopropyl, *n*-butyl, *tert*-butyl residues and combinations thereof;
and, provided that R₆ is selected from the group consisting of *n*-propyl, cyclopropyl, isobutyl, *tert*-butyl, neopentyl, and phenyl residues, R₄ and R₅ are not the same and are selected from the group consisting of methyl, ethyl, *n*-propyl, isopropyl, *n*-butyl, and *tert*-butyl residues; and
wherein, in the salts of formula (2), the product of said formula (2) is protonated.

Further, the present invention relates to a prodrug for use in the treatment of psychiatric disorders, the prodrug having the formula (2) or salts thereof, 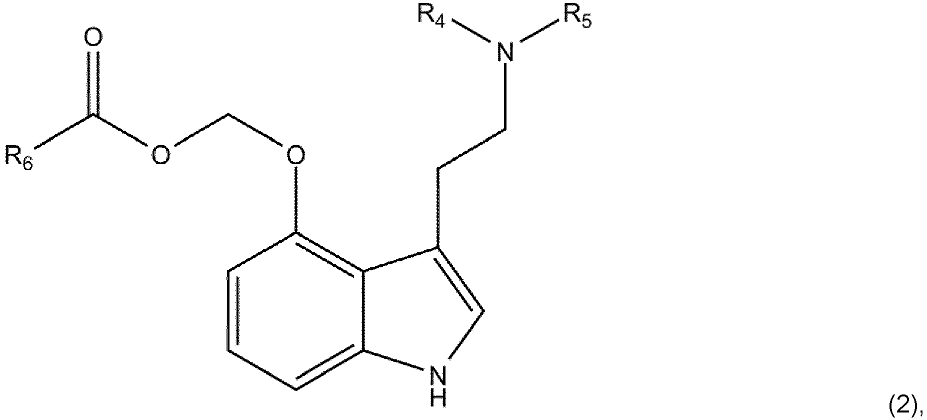
wherein R₆ is selected from the group consisting of cyclopropyl, isopropyl, *n*-propyl, isobutyl, *tert*-butyl, neopentyl, phenyl, and 2-furyl residues; and,
provided that R₆ is selected from the group consisting of isopropyl and 2-furyl residues, R₄ and R₅ are selected from the group consisting of methyl, ethyl, *n*-propyl, isopropyl, *n*-butyl, *tert*-butyl residues and combinations thereof;
and, provided that R₆ is selected from the group consisting of *n*-propyl, cyclopropyl, isobutyl, *tert*-butyl, neopentyl, and phenyl residues, R₄ and R₅ are not the same and are selected from the group consisting of methyl, ethyl, *n*-propyl, isopropyl, *n*-butyl, and *tert*-butyl residues; and
wherein, in the salts of formula (2), the product of said formula (2) is protonated.

The present inventors have conducted extensive research to provide a solution to the above problems and found that the herein described prodrugs and structures are excellently suited to be used in medicine and, particularly, in the treatment of psychiatric disorders.

In this work, the term "prodrug" relates to a pharmaceutically inactive compound that can be metabolized in the body to produce a pharmaceutically active drug. Regarding the herein shown derivatives of 4-hydroxytryptamines, the prodrugs are typically cleaved at the ester bond in the patient yielding the respective 4-hydroxytryptamine as the active compound.

Further, the term "psychedelic treatment" or "psychedelic therapy" relates to a treatment using psychedelic drugs or being assisted by psychedelic drugs, which is usually conducted under professional supervision.

Moreover, several forms of administering pharmaceuticals are mentioned hereinbelow. In this context, "buccal administration" refers to administration of a drug in the buccal area, i.e., in the cheek of a patient. "Sublingual administration" refers to the application of a drug under the tongue of a patient. Further, "inhalatory administration" refers to the application of a drug into the respiratory system of a patient by applying said drug during inhalation. Similarly, "intranasal administration" relates to administration of a drug into the nose of a patient. Further, the term "transdermal administration" refers to administration of a drug by delivering said drug across the skin of a patient.

Hereinafter, the prodrugs and their uses will be described in detail.

### Prodrugs having formula (1) - ester prodrugs

In a first aspect of the present invention, a prodrug is described. Said prodrug has the formula (1) or is a salt of a product of said formula (1).

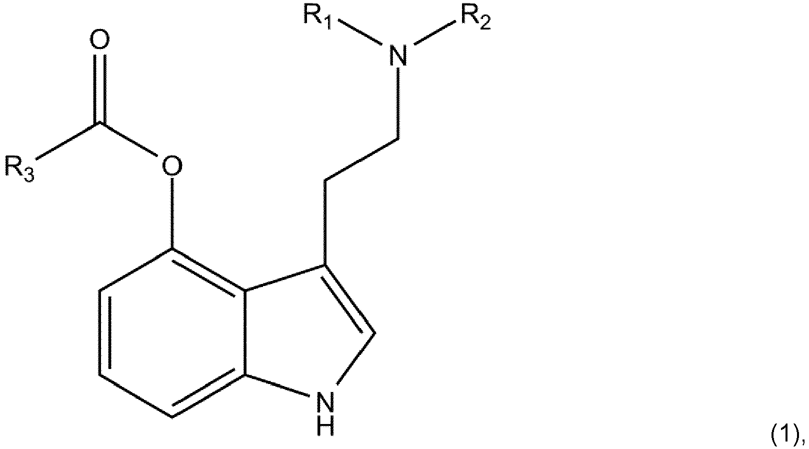

In formula (1), R₃ is selected from the group consisting of cyclopropyl, isopropyl, *n-*propyl, isobutyl, *tert*-butyl, neopentyl, phenyl, and 2-furyl residues. The choice of R₃ majorly impacts both the passive membrane permeability of the prodrug and its toxicity. Generally, high lipophilicity and high passive membrane permeability of the prodrugs inversely correlate with cell viability. Moreover, the nature of R₃ has a major impact on the cleavage rate of the herein described prodrugs in human saliva and human blood plasma. Therefore, R₃ has been chosen to accommodate for both a high passive membrane permeability and a low toxicity while also achieving a low cleavage rate in human saliva and a high cleavage rate in human blood plasma. Aliphatic promoieties comprising acyl residues with three to five, preferably three or four, carbon atoms along with aromatic promoieties represent a good compromise between the above-described factors and, particularly, between passive membrane permeability and cellular tolerability.

That is, in view of a good balance between high passive membrane permeability and cellular tolerability, R₃ is preferably selected from *n*-propyl, isopropyl, cyclopropyl, *tert-*butyl, isobutyl, phenyl and 2-furyl residues, i.e., each of said residues as well as any combination represents a particularly preferred embodiment of the present invention. Further, R₃ is particularly preferably selected from isopropyl, cyclopropyl, tert-butyl, and 2-furyl residues.

Further, in view of a low cleavage rate in human saliva of the herein described prodrugs, R₃ is preferably selected from *n*-propyl, isopropyl, 2-furyl, phenyl, cyclopropyl, and *tert-*butyl residues. Particularly preferred are isopropyl, 2-furyl, cyclopropyl, and *tert*-butyl residues, from which cyclopropyl and *tert*-butyl residues are most preferred in view of low cleavage rate in human saliva. It is to be noted that each of said residues as well as any combination represents a particularly preferred embodiment of the present invention.

Even further, in view of a high cleavage rate in human blood plasma of the herein described prodrugs, R₃ is preferably selected from *n*-propyl, isopropyl, cyclopropyl, phenyl, and 2-furyl residues. In this context, each of said residues as well as any combination represents a particularly preferred embodiment of the present invention. In this context, if R₃ is a *tert*-butyl residue, the cleavage rate in human blood plasma is low rendering the herein described prodrug in such case beneficial for applications requiring extended release in human blood plasma.

In a case that R₃ is selected from the group consisting of cyclopropyl, isopropyl, and 2-furyl residues, R₁ and R₂ are selected from the group consisting of methyl, ethyl, *n-*propyl, isopropyl, *n*-butyl, *tert*-butyl residues, and combinations thereof. However, in a specific embodiment, even in a case that R₃ is selected from the group consisting of cyclopropyl, isopropyl, and 2-furyl residues, R₁ and R₂ may not be the same and are selected from the group consisting of methyl, ethyl, *n*-propyl, isopropyl, *n*-butyl, and *tert*-butyl residues.

Further, in a case that R₃ is selected from the group consisting of *n*-propyl, isobutyl, *tert*-butyl, neopentyl, and phenyl residues, R₁ and R₂ are not the same and are selected from the group consisting of methyl, ethyl, *n*-propyl, isopropyl, *n*-butyl, and *tert*-butyl residues.

In view of the balancing of the above-described factors, i.e., the passive membrane permeability, toxicity, and optimal cleavage rates in saliva and plasma for a rapid onset, R₃ is preferably selected from the group consisting of cyclopropyl, isopropyl, *n*-propyl, 2-furyl, and phenyl residues and more preferably selected from the group consisting of cyclopropyl, isopropyl, 2-furyl, and phenyl residues. That is, in the case that R₃ is selected from the group consisting of cyclopropyl, isopropyl, *n*-propyl, and 2-furyl residues, R₁ and R₂ are preferably selected from methyl and ethyl residues. On the other hand, if R₃ is phenyl, preferably, R₁ is a methyl residue, and R₂ is an ethyl residue. Even more preferably, R₃ is selected from cyclopropyl and isopropyl residues, R₁ is a methyl residue, and R₂ is an ethyl residue.

Moreover, in the salts of formula (1), the product of said formula (1) is protonated. That is, the counterion of the herein described protonated compound is an anion.

The free bases of lipophilic tertiary amines, i.e., the prodrugs of formula (1) have a low solubility in water and usually have a liquid, viscous or resinous consistency at ambient conditions. These properties are not well compatible with the requirements of storage, purification, and pharmaceutical formulation of active pharmaceutical ingredients. Therefore, the herein described prodrugs may also be provided as salts, which improves water solubility significantly, rendering them more suitable for preparation procedures, storage, and most administration routes. On the other hand, providing the free bases is typically more effective for administration routes which require a certain degree of lipophilicity such as transdermal administration.

Preferably, an anion of the salts of formula (1) is selected from the group consisting of acetate, propionate, butyrate, malonate, maleate, succinate, levulinate, fumarate, mesylate, tartrate, citrate, lactate, pamoate, glutarate, adipate, and edisylate. More preferably, an anion of the salts of formula (1) is selected from the group consisting of acetate, propionate, butyrate, malonate, maleate, succinate, levulinate, fumarate, and mesylate and particularly, said anion is fumarate. That is, fumarates are particularly favorable since they form free-flowing, readily water-soluble colorless solids, thus, being optimal considering the above requirements of storage, purification, and pharmaceutical formulation. In this context, the term "fumarate" denotes a salt in which fumaric acid is completely deprotonated, thereby forming a salt with the compound of formula (1), which is monobasic, i.e., protonated once, in a 1:2 ratio. Thus, the alternative term "hemifumarate" may be used to indicate this stoichiometry. Importantly, the "fumarate" salt form has to be distinguished from other terms such as "hydrogen fumarate" or "bifumarate" which denote other protonation states and acid: base ratios.

As mentioned above, the prodrug of the present invention preferably has a high passive membrane permeability Pₑ. In this context, a passive membrane permeability is considered to be high if it is above 1.5 × 10⁻⁶ cm/s. Preferably, the passive membrane permeability Pₑ is at least 5 × 10⁻⁶ cm/s, at least 6 × 10⁻⁶ cm/s, more preferably at least 7 × 10⁻⁶ cm/s and even more preferably at least 8 × 10⁻⁶ cm/s. In particularly preferable cases, Pₑ may even be at least 9 × 10⁻⁶ cm/s, at least 10 × 10⁻⁶ cm/s, or even at least 11 × 10⁻⁶ cm/s. Particularly, a high Pₑ ensures that the prodrugs of the present invention are able to penetrate biological membranes without being dependent on active transport mechanisms. Thus, both the absorption of the prodrug in various tissues as well as the administration to the cells of interest are significantly affected by the passive membrane permeability.

In this context, comparing the 4-hydroxytryptamine parent compounds of the herein described prodrugs with the prodrugs of formula (1), esterification of the phenolic hydroxy group has an influence on the membrane permeability, i.e., it may increase or even decrease the membrane permeability. This may be attributed to the loss of an intramolecular hydrogen bond between said hydroxy group and the tertiary amino group. Such an intramolecular hydrogen bond can influence the polarity of the hydroxytryptamine by two mechanisms: First, by reducing the conformational flexibility of the hydroxytryptamine and thereby favoring conformations with a smaller polar surface area and less dipole momentum. Second, by reducing the number of possible hydrogen bonds of the involved functional groups to water molecules in the solvation shell. Thus, as mentioned above, specific residues R₃ of formula (1) are described here, which are sufficiently lipophilic to compensate for the loss of intramolecular hydrogen bonding and show a correlation with a high passive membrane permeability.

Here, the passive membrane permeability of various prodrugs was determined by means of a parallel artificial membrane permeability assay (PAMPA) as is described in detail below.

As has already been mentioned, a high passive membrane permeability has to be balanced with a low toxicity of the prodrugs, i.e., a suitable tolerance of human cells against the prodrugs. In this context, the prodrugs preferably have low toxicity towards human cells.

For example, if toxicity is tested using the human hepatocyte-derived HuH-7 cell line, a percentage of cells withstanding incubation using the prodrugs according to the present invention for 2 h (cell viability for 2 h) may be 60 % or more, preferably 70 % or more, and particularly preferably 80 % or more. Similarly, a percentage of cells withstanding incubation using the prodrugs according to the present invention for 24 h (cell viability for 24 h) may be 50 % or more, preferably 60 % or more, and particularly preferably 70 % or more. A suitable assay for evaluating cell viability is described in detail below.

Following absorption of the prodrugs by a patient, the temporal course of pharmacological drug effects is governed by the release kinetics of the prodrug in blood and potentially other tissues. In this context, blood represents the first and main compartment in which the prodrug is localized and from which it is distributed to the drug target sites. Within human blood, the herein described prodrugs are thought to be cleaved by plasma esterases or other plasma-specific factors.

In view of different applications of the prodrugs, both a fast or a slow cleavage of said prodrugs in human blood plasma might be beneficial. In particular, fast cleavage is preferred in cases where a fast drug effect is to be achieved. In contrast, in cases where a long-lasting drug effect is to be achieved, slow cleavage after administration is preferred. It is therefore possible, using the prodrugs described here, to adjust the surge and onset of the drug effects. In particular, a less sudden onset may be desired for a larger initial dose, whereas a faster onset may be preferred in a re-dosing setting during treatment.

Further to cleavage in human blood plasma, cleavage of the prodrugs in human saliva has a significant impact on suitability of said prodrugs for specific administration routes such as buccal and sublingual administration. That is, in order to achieve transport of a high amount of prodrug to a patient's blood and other tissues the cleavage rate of the prodrug in human saliva is preferably low. In this context, the term "low cleavage rate" relates to half-life periods of the prodrugs in human saliva of at least 15 min. In such time frames, the prodrug can readily be absorbed into the patient's tissues in short duration applications, e.g., buccal and sublingual applications, without excessive cleavage of the prodrug in the saliva prior to absorption.

In view of the above, the prodrug according to the present invention preferably exhibits a half-life period of at least 15 min in human saliva at 37 °C.

### Prodrugs having formula (2) - acyloxymethyl prodrugs

In a second aspect of the present invention, a prodrug having the formula (2) or salts of a product of formula (2) are provided.

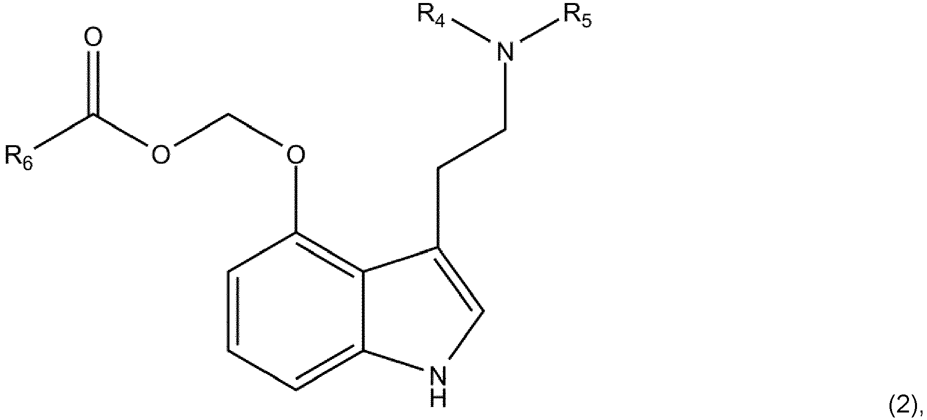

In formula (2), R₆ is selected from the group consisting of cyclopropyl, isopropyl, *n-*propyl, isobutyl, *tert*-butyl, neopentyl, phenyl, and 2-furyl residues. In a case that R₆ is selected from the group consisting of isopropyl and 2-furyl residues, R₄ and R₅ are selected from the group consisting of methyl, ethyl, *n*-propyl, isopropyl, *n*-butyl, *tert-*butyl residues and combinations thereof. However, in a specific embodiment, even in a case that R₆ is selected from the group consisting of isopropyl and 2-furyl residues, R₄ and R₅ may not be the same and are selected from the group consisting of methyl, ethyl, *n*-propyl, isopropyl, *n*-butyl, and *tert*-butyl residues.

Further, in a case that R₆ is selected from the group consisting of *n*-propyl, cyclopropyl, isobutyl, *tert*-butyl, neopentyl, and phenyl residues, R₄ and R₅ are not the same and are selected from the group consisting of methyl, ethyl, *n*-propyl, isopropyl, *n*-butyl, and *tert*-butyl residues. Moreover, similar to formula (1), in the salts of formula (2), the product of said formula (2) is protonated. That is, the counterion of the herein described protonated compound is an anion.

Further, regarding the salts of products of formula (2), the preferred salts described in relation to formula (1) are also applicable to said salts of the products of formula (2). Also, the preferred embodiments of the first aspect of the present invention fully apply to the prodrugs having formula (2) or the salts of products of formula (2).

Even further, the above explanations regarding the preferred passive membrane permeability, the toxicity, the cleavage and stability in human blood plasma and saliva also apply to the second aspect of the present invention and, of course, can be combined regarding both the first and the second aspect of the present invention.

### Synthesis method of acyloxymethyl derivatives of hydroxytryptamines

Further to the above-described prodrugs, a synthesis method of acyloxymethyl derivatives of hydroxytryptamines is provided.

In this context, in contrast to acid chlorides, which can be used to synthesize esters of the phenolic hydroxy of hydroxytryptamines such as e.g., compounds of formula (1), the softer alkyl halides required for acyloxymethylation are non-selective and react with the tertiary amino group and the indole nitrogen besides the phenolic hydroxy group. This leads to complex reaction mixtures and low yields of the desired product. Tedious and wasteful purification procedures afford the targeted acyloxymethyl prodrugs in generally less than 10 % yield (cf. e.g., WO 2023/023347 A1).

In general, the synthetic yields previously reported for the acyloxymethyl prodrugs (cf. WO 2023/023347 A1) are neither compatible with commercial production nor with an efficient exploration of the molecular diversity in this compound class. That is, synthetic yields of less than 10 % are incompatible with active pharmaceutical ingredient production, both from an economic and a pharmaceutical product quality perspective. A large extent of side reactions increases the risk of problematic contaminants in the final product, thus requiring wasteful and extensive purification procedures. Therefore, this aspect of the present invention provides a synthesis route having high and more reliable yield and being suitable for commercial production by addressing the described lack of chemoselectivity.

The herein described synthesis method of acyloxymethyl derivatives of hydroxytryptamines comprises the steps of
a) providing a hydroxytryptamine bearing a phenolic hydroxy group;
b) protecting the nitrogen comprised in the indole ring system of said hydroxytryptamine using a benzyloxycarbonyl protection group (Cbz);
c) reacting the *N*-protected hydroxytryptamine with a halogenomethyl carboxylate compound R'-(C=O)-OCH₂-Hal, thereby forming an *N*-protected acyloxymethyl hydroxytryptamine; and
d) deprotecting the nitrogen comprised in the indole ring system of the *N*-protected acyloxymethyl hydroxytryptamine.

In particular, the hydroxytryptamine provided in step a) comprises one phenolic hydroxy group without further hydroxy groups. Said hydroxytryptamine, for example, may comprise said phenolic hydroxy group at position 4 of the indole ring system. That is, said hydroxytryptamine may be psilocin or 4-hydroxy-*N*-methyl-*N*-ethyltryptamine (4-OH-MET).

The step b) of protecting the hydroxytryptamine can be achieved by reaction with the Heller-Sarpong reagent (formula (3), cf. Heller et al. Org. Lett. 2010, 12, 4572) under thermodynamic control, which yields an *N*-protected hydroxytryptamine in good to excellent yield. Said reaction can be conducted in anhydrous organic solvents, e.g., acetonitrile, using a non-nucleophilic base, e.g., 1,8-diazabicyclo[5.4.0]undec-7-ene (DBU), for instance at ambient temperature for several hours.

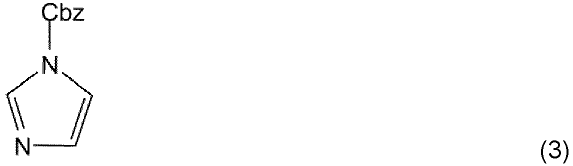

Subsequently, in step c), said *N*-protected hydroxytryptamine can be reacted with a halogenomethyl carboxylate compound R'-(C=O)-OCH₂-Hal. Due to the protection of the nitrogen comprised in the indole ring system, said compound is able to selectively react with the unprotected phenolic hydroxy group, thereby forming an *N*-protected acyloxymethyl hydroxytryptamine. An exemplary synthesis procedure is as follows. First, the *N*-protected hydroxytryptamine is treated with NaH in anhydrous *N,N*-dimethylformamide (DMF) at 0 °C until gas generation ceases. Then, the temperature is reduced to -30 to -50 °C and the halogenomethyl carboxylate compound R'-(C=O)-OCH₂-Hal is added in anhydrous tetrahydrofuran (THF). Subsequently, the reaction mixture is allowed to warm to ambient temperature while the reaction is continued for several hours. In view of it being an excellent leaving group, the halogen in step c) preferably is iodine.

In step d), the nitrogen comprised in the indole ring system is deprotected. Such deprotection step may be achieved e.g. by catalytic hydrogenation of the *N*-protected acyloxymethyl hydroxytryptamine using Pd/C and H₂.

The described synthesis procedure is able to provide acyloxymethyl hydroxytryptamines in high yields. That is, in the critical acyloxylmethylation step, isolated yields of e.g., 14 to 44 % are achieved, which represents a significant improvement as compared to the previously reported yields in the single-digit percentage range.

### Use of prodrugs having formula (1) or (2)

Further to the above, the present invention includes the use of a prodrug having formula (1) or (2) or salts of the products of said formulas (1) or (2) as a medicament. In this context, the preferred and particular embodiments described above are fully applicable regarding the use as a medicament.

In a specific use, the prodrug described herein can be used in the treatment of psychiatric and neurological disorders. Particularly, said disorders may be depression, post-traumatic stress disorder, substance use disorder, anorexia nervosa, and neurodegenerative diseases such as Alzheimer's disease.

Further, a particular treatment in which the prodrug can be applied includes psychedelic therapy.

Moreover, in view of the above-described use for treating psychiatric disorders, it is preferable that the prodrug's administration is buccal, sublingual, inhalatory, intranasal, and/or transdermal. Specifically, for instance, some of the above-described embodiments are particularly preferred in combination with specific drug administration routes, e.g., the free bases can be particularly advantageous for transdermal administration, which usually requires a certain degree of lipophilicity.

That is, specific pharmaceutical formulations that realize an advantageous pharmacokinetic profile in combination with the prodrugs described herein are, in particular, but not restricted to the following forms: lozenges, mucoadhesive patches or films, sprays for application in the oral cavity or in the nose, tablets and other solid dosage forms for buccal and sublingual application, pellets and other granular dosage forms for application in the oral or nasal cavities, liquid formulations for application in a nebulizer or vaporizer, semisolid or liquid dosage forms that increase their viscosity upon application in the oral or nasal cavity and form an adhesive film from which the absorption can occur. For the purpose of transdermal delivery, adhesive skin patches are one preferred formulation. In addition, semisolid dosage forms such as ointments or liquids and sprays which are applied to the skin can be used for transdermal application. Furthermore, the compounds described here may be applied in more conventional ways, such as in peroral solid, semisolid, or liquid dosage forms like tablets, hard capsules with a powdery, granular, or pellet filling, soft capsules with liquid, semisolid, or solid filling, suppositories and enemas.

Preferably, the prodrugs are not administered perorally or intravenously.

As has been mentioned above, avoiding peroral and intravenous administration and, preferably, providing buccal, sublingual, inhalatory, intranasal, and/or transdermal administration of the prodrugs can ensure a fast onset of the subjective effects during psychedelic treatment settings with lower inter-individual variability than oral psilocybin dosage. Thus, undesired waiting times can be eliminated, the efficiency of therapist interactions can be increased, and the predictability of the evoked subjective experience can be improved. Further, a dosing with fast onset allows the adjustment of the psychedelic experience.

Even further, intravenous or other invasive and potentially threatening forms of drug administration may be incompatible with the intimate and preferentially non-clinical treatment setting, particularly, if the prodrugs are applied in psychedelic therapy.

Moreover, considering that some psychiatric disorders may involve restricted or absent swallowing capacity of patients, as in Alzheimer's disease, application routes of the prodrugs are required that do not rely on the patient's ability to swallow. In such cases, the administration cannot reasonably be peroral. That is, in this specific case, the most preferable administration may be transdermal.

An additional advantage of bypassing the peroral and gastrointestinal administration route is that the effects of first-pass metabolism in the liver are eliminated. Thereby, it may be possible to use lower dosages. Furthermore, metabolism in the liver has individual variability due to induction or inhibition of enzymes and other predispositions such as age and the presence or absence liver diseases. If the first-pass metabolism is avoided by non-enteral application routes, a more reliable and less variable effect of the applied drug can be expected.

### Examples

The present invention is further described in the following with reference to examples.

### Materials and methods

Chemicals and solvents were purchased from commercial suppliers such as Carbolution, BLDpharm, Fisher Scientific, Sigma Aldrich, and TCI. Ethyl acetate was distilled prior to use. All other chemicals were used as received if not stated otherwise. Psilocin (4-OH-DMT, 1) was prepared based on literature procedures (cf. Sherwood et al. Synthesis 2020, 52, 688; and Shirota et al. J. Nat. Prod. 2003, 66, 885). For purification and analytic characterizations, the following devices and methods were used:

### Amberlyst A-21 activation

Commercial Amberlyst A-21 resin (Sigma-Aldrich, Germany) was activated by adding HPLC-grade methanol (1 mL per gram resin), stirring for 5 min, and filtration. This treatment was repeated with anhydrous THF (2x0.8 mL per gram resin) and HPLC-grade dichloromethane (DCM) (2x0.8 mL per gram resin). Volatiles were removed by rotary evaporation, and the resin was further dried under high vacuum overnight to give a free-flowing solid that was stored in an amber-colored bottle.

### Column Chromatography

Flash column chromatography was carried out on silica gel (40 - 63 µm, 60 Å, Material Harvest) using a Biotage Isolera One system and various column sizes. The dimensions of the columns are provided in the detailed synthetic descriptions below.

### Lyophilization

Freeze-drying was performed on a Christ Alpha 1-2 LDplus lyophilizer.

### High-performance liquid chromatography (HPLC)

Analytical measurements were performed using a Jasco HPLC system equipped with an RP-18 column. Detailed information on chromatographic separation and detection is given in the description of the respective application.

Preparative HPLC was performed on an ÄKTA purifier (GE Healthcare, Germany) equipped with a Reprospher 100 C18-DE guard column (5 µm, 30 × 16 mm, Dr. Maisch GmbH) and a Reprosil 100 C18 column (10 µm, 150 × 30 mm, Dr. Maisch GmbH). Mixtures of distilled water (eluent A) and acetonitrile (eluent B) buffered with 0.1 % trifluoroacetic acid (TFA) were used as mobile phase at a flow rate of 20.0 mL/min using one of the gradients listed in Table 1. The chromatographic separation was monitored by UV detection at 214, 254, and 280 nm.

**Table 1: Gradient settings.**

| gradient | settings |
|---|---|
| **I** | 30 % B (0.6 CV); 100 % B (4.6 CV); 100 % B (6.1 CV); 30 % B (6.2 CV); 30 % B (7.2 CV) |
| **II** | 10 % B (0.6 CV); 100 % B (4.6 CV); 100 % B (6.1 CV); 10 % B (6.2 CV); 10 % B (7.2 CV) |

### Mass Spectrometry

High resolution mass spectra (HR MS) were recorded on a Bruker micrOTOF-Q II mass spectrometer calibrated with sodium formate cluster ions.

### NMR Spectroscopy

¹H, ¹³C, and ¹⁹F NMR spectra as well as APT spectra were recorded at room temperature on 300 MHz or 500 MHz Varian instruments. In addition, COSY, HSQC and HMBC spectra were recorded but are not shown in this application for the sake of brevity. Chemical shifts (δ) are reported in parts per million (ppm) relative to the residual undeuterated or partially deuterated solvent peak according to the literature (cf. Fulmer et al. Organometallics 2010, 29, 2176). Coupling constants (J) are reported in Hertz (Hz). The following abbreviations are used to indicate the signal multiplicity: s (singlet), d (doublet), t (triplet), q (quartet), quin (quintet), sxt (sextet), spt (septet), oct (octet), n (nonet), m (multiplet), br s (broad singlet) as well as their combinations. ¹³C signals were classified as follows: C_{q} (quarternary), CH (tertiary), CH₂ (secondary), and CH₃ (primary). All NMR spectra were integrated and processed using ACD/Spectrus Processor 2016.1.

### pH Measurements

The pH values of aqueous solutions were measured using a pH meter 766 (Knick, Germany).

### Thin-layer Chromatography (TLC)

TLC was performed on Macherey-Nagel Polygram^{®} SIL G/UV₂₅₄ and ALOX N/UV₂₅₄ precoated sheets. Components were visualized by UV irradiation (254 nm) or by staining with aqueous KMnO₄ solution (1 g KMnO₄, 2 g Na₂CO₃, 100 mL H₂O) or aqueous FeCl₃ solution (1 g FeCls, 50 mL H₂O, 50 mL MeOH).

### Passive membrane permeation assay (PAMPA)

The passive membrane permeability was evaluated with a pre-coated PAMPA plate system (BD Gentest, BD Bioscience, Germany) following the guidelines of the manufacturer and a previous literature report (cf. Kühl et al. Eur. J. Med. Chem. 2022, 240, 114585). Phosphate-buffered saline (PBS) pH 7.4 (Sigma-Aldrich, Germany) was used for all experiments. Analyte concentrations were determined on a Jasco HPLC system equipped with a UV detector and an RP-18 column (Chromolith^{®} Performance RP-18e, 100x4.6 mm) using one of the gradients listed in Table 2. For all samples, the injection volume was 40 µl.

**Table 2: HPLC settings for the analysis of PAMPA samples.**

| **Eluent A** | **Eluent B** | **Flow** | **Gradient** |
|---|---|---|---|
| water (0.1 % TFA) | acetonitrile (0.1 % TFA) | 1.0 mL/min | 1 % B (0.2 min) |
| | | | 100 % B (7.0 min) |
| | | | 100% B (8.0 min) |
| | | | 1 % B (8.1 min) |
| | | | 1 % B (10.0 min) |
| water (0.1 % TFA) | acetonitrile (0.1 % TFA) | 1.0 mL/min | 1 % B (0.2 min) |
| | | | 50 % B (7.0 min) |
| | | | 50 % B (8.0 min) |
| | | | 1 % B (8.1 min) |
| | | | 1 % B (10.0 min) |

UV detection was performed at 286 nm except for phenytoin (220 nm) and acetyl salicylic acid (228 nm). Prior to performing the assay, the PAMPA plate was warmed to room temperature for 0.5 h. In the donor plate, 300 µL of the 200 µM compound solutions in PBS (4 % dimethyl sulfoxide (DMSO)) were dispensed in triplicate, and in the acceptor plate, 200 µL of PBS buffer were added to all wells. The plates were combined, and the system was incubated at 25 °C for 5 h. Upon completion of the incubation, donor and acceptor plate were separated. The wells were homogeneously mixed with a pipette, and a 100 µl sample of each donor and acceptor well was transferred to 96 well U-bottom polypropylene plates (Greiner Bio-One, Germany) for quantitative HPLC analysis. Six-point calibration curves (10, 25, 50, 100, 150, and 200 µM) were generated for all permeating compounds and references (Phenytoin, Imipramine HCl, Caffeine) with correlation coefficients (R²) being at least 0.99. For Furosemide, an eight-point calibration curve (0.1, 1, 10, 25, 50, 100, 150, and 200 µM) was generated due to the low passive membrane permeability. Permeability (Pₑ) and mass retention (R) were calculated as described in the literature (cf. e.g., Chen et al. Pharm. Res. 2008, 25, 1511; and Kansy et al. J. Med. Chem. 1998, 41, 1007).

### Stability in phosphate-buffered saline

Accelerated degradation studies were performed using Dulbecco's phosphate buffered saline (PBS) pH 7.3 (Sigma-Aldrich, Germany). In a thermoblock, 1386 µL PBS and 28 µl of a 5 mM analyte stock solution in DMSO were prewarmed to 80 °C for 10 min in separate plastic vials. Then, 14 µl of the prewarmed 5 mM analyte stock solution were added to the prewarmed PBS, and the mixture was vortexed vigorously. In appropriate time intervals, samples of the reaction mixture (100 µl) were taken directly after vortexing. The samples were kept at -20 °C until being analyzed or analyzed directly. Analyte quantifications were performed on a Jasco HPLC system equipped with a UV detector and an RP-18 column (Chromolith^{®} Performance RP-18e, 100x4.6 mm) using one of the acetonitrile/water gradients listed in Table 2. For all samples, the injection volume was 40 µl and UV detection was performed at 286 nm.

The data were analyzed graphically (Origin Pro 2015) assuming zero and first order kinetics. Data points with an area under the curve (AUC) of less than 100 µV·min were not included in further analysis due to their insufficient signal-to-noise ratio. In all cases, first order kinetics were found as judged by the quality of fit (R²>0.9). Errors were estimated based on the uncertainty of the linear fit.

### Cell viability

Cell viability in HuH-7 cells in the presence of compound dilutions was determined using the CellTiter-Blue reagent (Promega) according to manufacturer's instructions (cf. CellTiter-Blue Cell Viability Assay, Promega Corporation, Madison, 2016). In order to assess cytotoxic effects of compounds in cell culture, HuH-7 cells were incubated in presence of these compounds in a 96-well plate for 2 h or 24 h. In order to generate a calibration curve for cell viability, a serial dilution (1:2 dilution series) of cells was seeded into control wells. Measurement of cell viability is based on the assay development using CellTiter-Blue reagent (Resazurin, blue color), which is reduced to resofurin (pink color) by viable cells.

Prior to performing the assay, a 96-well assay plate (F-bottom, transparent, Greiner) containing HuH-7 cells (20,000 cells per well) in culture medium was prepared. Test compounds were added to obtain a final volume of 100 µL per well before the 96-well plate was incubated at 37 °C, 5 % CO₂ and 95 % relative humidity (RH) for 2 h or 24 h. CellTiter-Blue reagent (1-2 mL) was diluted with cell culture medium (10 mL) in a Falcon tube and prewarmed to 37 °C. The 96-well plate was removed from the incubator, the medium aspirated using a vacuum pump and 100 µL of diluted CellTiter-Blue reagent was added to each well. After incubation at 37 °C, 5 % CO₂ and 95 % RH for 1-3 h, a color change was visible and the fluorescence was measured at 560/590 nm (λₑₓ/λₑₘ).

### Drug release kinetics in human blood plasma

The release kinetics of the prodrugs were evaluated *in vitro* in analogy to the precedent literature (cf. e.g., Christrup et al. Int. J. Pharm. 1997, 154, 157). As ZnSO₄ was found to affect the analyte's signal intensity presumably by complexation, acetonitrile (2:1) was used as protein precipitation agent (cf. Polson et al. J. Chromatogr. B 2003, 785, 263). Pooled human blood plasma (Biowest, France) and sterile Dulbecco's phosphate buffered saline (PBS) pH 7.3 (Sigma-Aldrich, Germany) were used for all experiments. The blood plasma was aliquoted upon receipt and stored at -20 °C. 10 % solutions were prepared by thawing a plasma aliquot at 2-8 °C, distributing it into plastic vials and diluting with the appropriate volume of sterile PBS. The 10 % and 100 % plasma aliquots were stored at -20 °C and thawed at 2-8 °C directly before the assay was performed. In a thermoblock, a plasma aliquot and 10 µl of a 5 mM prodrug solution in DMSO were prewarmed to 37 °C for 10 min in separate plastic vials. Then, 1000 µl of the prewarmed plasma sample was added to the prodrug solution, and 9×100 µl samples were withdrawn at appropriate time intervals. The enzymatic reaction was quenched by immediate mixing of the samples (100 µl) with acetonitrile (200 µl). Blank samples were prepared by mixing 10 % or 100 % plasma (100 µl) with acetonitrile (200 µl), respectively. Samples of the initial reaction mixture were prepared by adding the 5 mM prodrug solution (1 µl) to a blank sample.

Centrifugation (13,000 rpm, 3 min) of the quenched samples afforded clear supernatants which were transferred to 96 well U-bottom polypropylene plates (Greiner Bio-One, Germany). Analyte quantifications were performed on a Jasco HPLC system equipped with a UV detector and an RP-18 column (Chromolith^{®} Performance RP-18e, 100x4.6 mm) using one of the gradients listed in Table 3. Prodrug degradation was monitored using undiluted supernatant and a 10 min chromatographic method. Simultaneous quantification of residual prodrug and released parent drug was performed with diluted samples (75 µl supernatant, 25 µl H₂O (+0.4 % TFA)) using a 17 min method. For all samples, the injection volume was 40 µL and the flow was set to 1.0 mL/min. UV detection was performed at 286 nm except for acetyl salicylic acid (ASA, 228 nm). The data were analyzed graphically (Origin Pro 2015) assuming zero and first order kinetics. If not stated otherwise, first order kinetics were found as judged by the quality of fit (R²>0.9).

**Table 3: HPLC settings for quantitative analysis of plasma samples.**

| **Analyte** | **Eluent A** | **Eluent B** | **Gradient** |
|---|---|---|---|
| **8a, 8e** | water (0.1 % TFA) | acetonitrile (0.1 % TFA) | 1 % B (0.2 min) |
| | | | 30 % B (7.0 min) |
| | | | 30 % B (8.0 min) |
| | | | 1 % B (8.1 min) |
| | | | 1 % B (10.0 min) |
| **ASA** | water (0.1 % TFA) | acetonitrile (0.1 % TFA) | 1 % B (0.2 min) |
| | | | 50 % B (7.0 min) |
| | | | 50 % B (8.0 min) |
| | | | 1 % B (8.1 min) |
| | | | 1 % B (10.0 min) |
| **8b-d, 8g-i** | water (0.1 % TFA) | acetonitrile (0.1 % TFA) | 1 % B (0.2 min) |
| **10, 11** | | | 100 % B (7.0 min) |
| **15a-d** | | | 100% B (8.0 min) |
| | | | 1 % B (8.1 min) |
| | | | 1 % B (10.0 min) |
| **7a, 7b** | water (0.1 % TFA) | acetonitrile (0.1 % TFA) | 1 % B (0.2 min) |
| **8b** | | | 20 % B (10.0 min) |
| | | | 30 % B (10.1 min) |
| | | | 30 % B (15 min) |
| | | | 1 % B (15.1 min) |
| | | | 1 % B (17.0 min) |
| **8i, 15b** | water (0.1 % TFA) | acetonitrile (0.1 % TFA) | 1 % B (0.2 min) |
| | | | 20 % B (10.0 min) |
| | | | 35 % B (10.1 min) |
| | | | 35 % B (15 min) |
| | | | 1 % B (15.1 min) |
| | | | 1 % B (17.0 min) |
| **15d** | water (0.1 % TFA) | acetonitrile (0.1 % TFA) | 1 % B (0.2 min) |
| | | | 20 % B (10.0 min) |
| | | | 40 % B (10.1 min) |
| | | | 40 % B (15 min) |
| | | | 1 % B (15.1 min) |
| | | | 1 % B (17.0 min) |
| **8c** | water (0.1 % TFA) | acetonitrile (0.1 % TFA) | 1 % B (0.2 min) |
| | | | 20 % B (10.0 min) |
| | | | 45 % B (10.1 min) |
| | | | 45 % B (15 min) |
| | | | 1 % B (15.1 min) |
| | | | 1 % B (17.0 min) |

### Prodrug stability in human whole saliva

Human whole saliva was collected from three non-smoking individuals (1 male, 2 female, age 24-27 years) by expectoration into plastic vials (spitting method), which has been recommended in the literature (cf. Navazesh, Ann. N. Y. Acad. Sci. 1993, 694, 72). Sampling was performed before breakfast, i.e. at least 8 h after the last food intake and tooth brushing, in order to minimize the impact of diet and dental materials on esterase activity (cf. Lindqvist et al. Eur. J. Oral Sci. 1980, 88, 229).

Freshly collected saliva and 5 mM stock solutions of the analytes in DMSO were prewarmed to 37 °C for 10 min. Then, 700 µl prewarmed saliva was added to 7 µl of the prodrug stock, and the mixture was incubated at 37 °C. Samples (100 µL) were taken at selected time points (0, 10, 20, 40, 60 min; 2 h or 24 h) and immediately quenched with acetonitrile (200 µl). Blank samples were prepared by mixing saliva (100 µl) with acetonitrile (200 µl). Centrifugation (3,000 rpm, 5 min) of the quenched samples afforded clear supernatants which were transferred to 96 well U-bottom polypropylene plates (Greiner Bio-One, Germany). Analyte quantifications were performed on a Jasco HPLC system equipped with a UV detector and an RP-18 column (Chromolith^{®} Performance RP-18e, 100x4.6 mm) using the gradients listed in Table 4. Samples that gave rise to asymmetric peaks inadequate for quantification were diluted (1:1) with H₂O (+0.1 % TFA). The injection volume was 40 µl and the flow was set to 1.0 mL/min. UV detection was performed at 286 nm. The data were analyzed graphically (Origin Pro 2015) assuming zero and first order kinetics. If not stated otherwise, the reactions followed first order kinetics as judged by the quality of fit (R²>0.9).

**Table 4: HPLC settings for quantitative analysis of saliva samples.**

| **Analyte** | **Eluent A** | **Eluent B** | **Gradient** |
|---|---|---|---|
| **8b, 8d, 8f, 8i** | water (0.1 % TFA) | acetonitrile (0.1 % TFA) | 1 % B (0.2 min) |
| **15a, 15b, 15d** | | | 100 % B (7.0 min) |
| | | | 100% B (8.0 min) |
| | | | 1 % B (8.1 min) |
| | | | 1 % B (10.0 min) |
| **7a, 7b** | water (0.1 % TFA) | acetonitrile (0.1 % TFA) | 1 % B (0.2 min) |
| **15c** | | | 50 % B (7.0 min) |
| | | | 50 % B (8.0 min) |
| | | | 1 % B (8.1 min) |
| | | | 1 % B (10.0 min) |
| **8c** | water (0.1 % TFA) | acetonitrile (0.1 % TFA) | 1 % B (0.2 min) |
| | | | 100% B (12.0 min) |
| | | | 100% B (13.0 min) |
| | | | 1 % B (13.1 min) |
| | | | 1 % B (15.0 min) |
| **7c,8e** | water (0.1 % TFA) | acetonitrile (0.1 % TFA) | 1 % B (0.2 min) |
| | | | 30 % B (7.0 min) |
| | | | 30 % B (8.0 min) |
| | | | 1 % B (8.1 min) |
| | | | 1 % B (10.0 min) |
| **8a** | water (0.1 % TFA) | acetonitrile (0.1 % TFA) | 1 % B (0.2 min) |
| | | | 25 % B (7.0 min) |
| | | | 25 % B (8.0 min) |
| | | | 1 % B (8.1 min) |
| | | | 1 % B (10.0 min) |

### Syntheses of precursors

### 3-(2-(Ethyl(methyl)amino)-2-oxoacetyl)-1H-indol-4-yl acetate (18)

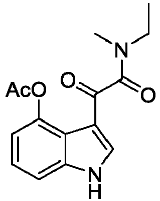

To an ice-cooled solution of oxalyl chloride (881 µl, 1.30 g, 10.3 mmol, 1.80 eq) in anhydrous methyl *tert*-butyl ether (MTBE) (3.3 mL) under inert atmosphere was added 4-acetoxyindole (1.00 g, 5.71 mmol, 1.00 eq) dissolved in anhydrous MTBE (6.7 mL) dropwise over 5 min. While stirring for 4 h at 0-10°C, a bright yellow precipitate formed. Then, volatiles were removed *in vacuo,* and the residue was dissolved in anhydrous THF (16.5 mL). The obtained solution was cooled with an icebath, and a solution of N-ethyl-*N*-methylamine (1.18 mL, 812 mg, 13.7 mmol, 2.40 eq) in anhydrous THF (2.7 mL) was added dropwise. Subsequently, the reaction mixture was allowed to warm to room temperature and stirred overnight. Upon completion, volatiles were removed by rotary evaporation and the residue was dissolved in DCM (200 mL). The organic phase was washed with 0.1 N HCl (2x50 mL), brine (2x50 mL), dried over anhydrous Na₂SO₄, and evaporated. The crude was finally recrystallized from a mixture of DCM (1.5 mL) and 2-propanol (6.0 mL), filtered over a Büchner funnel with filter paper, and washed with heptane (2.0 mL), which afforded **18** (1.15 g, 3.98 mmol, 69 %) as an off-white solid.

The NMR spectra show a mixture of amide rotamers (Rotamer 1: Rotamer 2 = 1:1.3). Rotamer 1: **¹H NMR (500 MHz, (CD₃)₂SO):** δ = 12.34-12.66 (m, 1H), 8.05 (d, *J* = 3.2 Hz, 1H), 7.40-7.49 (m, 1H), 7.29 (td, *J* = 7.9 Hz, *J'* = 1.4 Hz, 1H), 6.92 (d, *J* = 7.6 Hz, 1H), 3.43 (q, *J* = 7.1 Hz, 2H), 2.83 (s, 3H), 2.27-2.41 (m, 3H), 1.14 (t, *J* = 7.2 Hz, 3H) ppm. Rotamer2: **¹H NMR (500 MHz, (CD₃)₂SO):** δ = 12.34-12.66 (m, 1H), 8.11 (d, *J* = 3.3 Hz, 1H), 7.40-7.49 (m, 1H), 7.29 (td, *J* = 7.9 Hz, *J'* = 1.4 Hz, 1H), 6.92 (d, *J* = 7.6 Hz, 1H), 3.19 (q, *J* = 7.0 Hz, 2H), 2.93 (s, 3H), 2.27-2.41 (m, 3H), 1.05 (t, *J* = 6.97 Hz, 3H) ppm. **¹³C NMR (75 MHz, CDCl₃):** δ = 185.6 (C_{q}, 1C), 171.1/170.9 (C_{q}, 1C), 168.6/168.1 (C_{q}, 1C), 144.4 (C_{q}, 1C), 139.3 (C_{q}, 1C), 138.1 (CH, 1C), 124.8 (CH, 1C), 118.4 (C_{q}, 1C), 116.1/116.0 (CH, 1C), 113.7/113.6 (C_{q}, 1C), 110.9 (CH, 1C), 45.1/41.7 (CH₂, 1C), 34.9/31.4 (CH₃, 1C), 21.7 (CH₃, 1C), 13.5/12.1 (CH₃, 1C) ppm. **HR MS (ESI⁺):** m/z [2M+Na]⁺ calcd. for [C₃₀H₃₂N₄NaO₈]⁺: 599.2112, found 599.2113; [M+Na]⁺ calcd. for [C₁₅H₁₆N₂NaO₄]⁺: 311.1002, found 311.1010.

### 4-Hydroxy-N-ethyl-N-methyltryptamine (6)

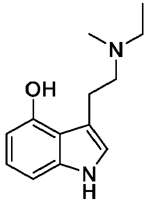

Under inert atmosphere, **18** (1.50 g, 5.20 mmol, 1.00 eq) was dissolved in anhydrous 2-Me-THF (47 mL) and cooled to 0°C by immersing the Schlenk tube into an ice bath. Then, a 2.3 M solution of LiAlH₄ in 2-Me-THF (7.24 mL, 632 mg, 16.7 mmol, 3.2 eq) was added dropwise by syringe yielding a bright yellow suspension. After the addition, the ice bath was removed, a nitrogen-filled balloon was installed, and the mixture was heated to reflux. After refluxing for 4 h, the reaction mixture was cooled with an ice bath, and quenched by dropwise addition of THF/H₂O (27:100, 1.73 mL). Anhydrous Na₂SO₄ (3.45 g) was added, followed by aminofunctionalized silica gel (1.71 g) and DCM (14.4 mL), and the mixture was stirred for 15 min before being filtered via a fritted glass piece under inert atmosphere. The filter cake was washed with a DCM/MeOH mixture (9:1, 55 mL), and the combined filtrates were concentrated by rotary evaporation to give a yellow solid. This was filtered through a pad of aminofunctionalized silica (m= 9.829 g) using DCM (492 mL) as eluent. Rotary evaporation finally afforded the title compound **6** (874.4 mg, 4.01 mmol, 77 %) as a colorless crystalline solid. **¹H NMR (500 MHz, CD₃CN):** δ = 12.92 (br s, 1H), 8.90 (br s, 1H), 6.91 (t, *J* = 7.9 Hz, 1H), 6.89 (d, *J* = 2.2 Hz, 1H), 6.82 (dd, *J* = 8.1 Hz, *J'* = 0.9 Hz, 1H), 6.32 (dd, *J* = 7.5 Hz, *J'* = 0.9 Hz, 1H), 2.86-2.93 (m, 2H), 2.63-2.70 (m, 2H), 2.51 (q, *J* = 7.2 Hz, 2H), 2.30 (s, 3H), 0.98 (t, *J* = 7.2 Hz, 3H) ppm. **¹³C NMR (126 MHz, CD₃CN):** δ = 153.2 (C_{q}, 1C), 140.2 (C_{q}, 1C), 123.7 (CH, 1C), 122.4 (CH, 1C), 118.7 (C_{q}, 1C), 114.9 (C_{q}, 1C), 106.4 (CH, 1C), 103.4 (CH, 1C), 60.1 (CH₂, 1C), 52.7 (CH₂, 1C), 42.4 (CH₃, 1C), 26.0 (CH₂, 1C), 11.7 (CH₃, 1C) ppm. **HR MS (ESI⁺):** m/z [M+Na]⁺ calcd. for [C₁₃H₁₈N₂NaO]⁺: 241.1311, found 241.1313.

### 4-Hydroxy-N-ethyl-N-methyltryptammonium fumarate (17)

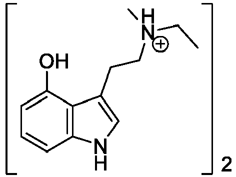

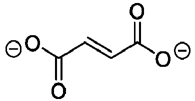

The free base **6** (95.1 mg, 436 µmol, 1.00 eq) was dissolved in acetone (1.5 mL), mixed with a solution of fumaric acid (25.3 mg, 218 µmol, 0.50 eq) in acetone (4.0 mL), and allowed to stand at 5 °C for 1.5 h. Finally, the suspension was centrifuged (3,500 RCF, 5 min), the supernatant decanted, and the solids washed twice with cold acetone (5 °C, 2x1.2 mL), taken up in distilled water and lyophilized. This afforded **17** (106 mg, 383 µmol, 88 %) as a colorless solid.

**¹H NMR (500 MHz, (CD₃)₂SO):** δ = 11.51 (br s, 1H), 10.64 (br s, 1H), 6.95 (d, *J* = 2.2 Hz, 1H), 6.80 (t, *J* = 7.7 Hz, 1H), 6.75 (dd, *J* = 7.9 Hz, *J' =* 1.0 Hz, 1H), 6.48 (s, 1H), 6.28 (dd, *J* = 7.5 Hz, *J*' = 0.9 Hz, 1H), 2.97 (t, *J* = 7.1 Hz, 2H), 2.84 (t, *J* = 7.0 Hz, 2H), 2.69 (q, *J* = 7.1 Hz, 2H), 2.42 (s, 3H), 1.06 ppm (t, *J* = 7.2 Hz, 3H) ppm. **¹³C NMR (126 MHz, (CD₃)₂SO):** δ = 167.5 (C_{q}, 1C), 151.6 (C_{q}, 1C), 138.7 (C_{q}, 1C), 134.9 (CH, 1C), 121.9 (CH, 1C), 121.3 (CH, 1C), 116.7 (C_{q}, 1C), 111.6 (C_{q}, 1C), 103.4 (CH, 1C), 102.8 (CH, 1C), 57.7 (CH₂, 1C), 50.4 (CH₂, 1C), 40.5 (CH₃, 1C), 23.2 (CH₂, 1C), 10.6 (CH₃, 1C) ppm. **HR MS (ESI⁺):** m/z [2M+C₂H₂]²⁺ calcd. for [C₂₈H₃₈N₄O₂]²⁺: 231.1492, found 231.1486; [M+H]⁺ calcd. for [C₁₃H₁₉N₂O]⁺: 219.1492, found 219.1489. The spectroscopic data correspond to the ones previously reported in the literature.^{[30]}

### Synthesis of ester prodrugs having formula (1)

### General procedure (GP1): HPLC-pure material for biological assays

Under inert atmosphere, the 4-hydroxytryptamine (1.00 eq) was dissolved in anhydrous THF (0.5 M with respect to the 4-hydroxytryptamine), and the solution was cooled to 0 °C. Subsequently, the respective acid chloride was added. The resulting suspension was allowed to warm to ambient temperature and stirred until the starting material was completely consumed according to TLC. Unreacted starting material was visualized by staining with FeCl₃ solution. Upon completion, volatiles were removed by rotary evaporation, and the obtained crude was purified by means of reversed-phase chromatography on a preparative HPLC instrument (gradient II). The collected product fractions were concentrated by rotary evaporation and lyophilized.

The obtained residue was dissolved in DCM (75 mM with respect to the tryptamine) and stirred with activated Amberlyst A-21 (0.87 mg Amberlyst A-21 per µmol tryptamine) for 30 min, filtered and concentrated. This procedure was carried out four times in order to ensure quantitative trifluoroacetate removal. Alternatively, the oily residue was partitioned between diethyl ether and carbonate buffer (0.1 M, pH 10.6), washed twice with carbonate buffer, dried over Na₂SO₄ and evaporated. The generated free base (1.00 eq) was then dissolved in acetone (at least 137 mM with respect to the tryptamine), mixed with a solution of fumaric acid (0.50 eq) in acetone (at least 46 mM with respect to fumaric acid), and allowed to stand at 5 °C for at least 1.5 h. Finally, the suspension was centrifuged (3,500 RCF, 5 min), the supernatant decanted, and the solids washed twice with cold acetone (5 °C), taken up in distilled water and lyophilized. If required, residual traces of acetone were removed by heating to 40 °C for 22 h.

An exemplary procedure for the synthesis and purification on the gram scale is provided in the description of compound **7c.**

### 4-iso-Butyryloxy-N,N-dimethyltryptammonium fumarate (7a)

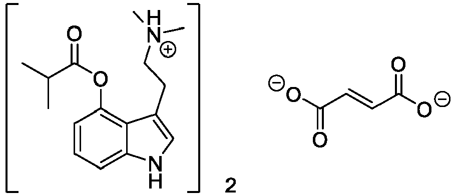

4-Hydroxy-*N,N*-dimethyltryptamine **1** (199.9 mg, 979 µmol, 1.00 eq) was reacted with iso-butyryl chloride (105 µl, 104 mg, 979 µmol, 1.00 eq) for 5 h, purified by HPLC and subsequently converted into 4-*iso*-butyryloxy-*N*,*N*-dimethyltryptamine (**19a**) by alkaline extraction as described in **GP1. 19a** (200 mg, 730 µmol, 75 %) was obtained as a yellow oil.

**¹H NMR (500 MHz, (D₃C)₂CO):** δ = 10.15 (br s, 1H), 7.25 (d, *J* = 8.1 Hz, 1H), 7.15 (s, 1H), 7.05 (t, *J* = 7.9 Hz, 1H), 6.68 (d, *J* = 7.6 Hz, 1H), 2.95 (spt, *J* = 7.0 Hz, 1H), 2.85-2.90 (m, 2H), 2.48-2.57 (m, 2H), 2.23 (s, 6H), 1.36 (d, *J* = 7.0 Hz, 6H) ppm. **¹³C NMR (126 MHz, (D₃C)₂CO):** δ = 176.1 (C_{q}, 1C), 145.6 (C_{q}, 1C), 140.0 (C_{q}, 1C), 123.8 (CH, 1C), 122.0 (CH, 1C), 121.2 (C_{q}, 1C), 113.3 (C_{q}, 1C), 112.5 (CH, 1C), 109.9 (CH, 1C), 62.0 (CH₂, 1C), 45.8 (CH₃, 2C), 34.9 (CH, 1C), 26.0 (CH₂, 1C), 19.3 (CH₃, 2C) ppm. **HR MS (ESI⁺):** m/z [M+Na]⁺ calcd. for [C₁₆H₂₂N₂NaO₂]⁺: 297.1573, found 297.1574.

Reaction of **19a** (200 mg, 730 µmol, 1.00 eq) with fumaric acid according to GP1 yielded **7a** (240 mg, 721 µmol, 99 %) as a colorless solid.

**¹H NMR (500 MHz, (D₃C)₂SO):** δ = 11.09 (br s, 1H), 7.23 (d, *J* = 8.1 Hz, 1H), 7.16 (d, *J* = 1.2 Hz, 1H), 7.03 (t, *J* = 7.9 Hz, 1H), 6.63 (d, *J* = 7.6 Hz, 1H), 6.52 (s, 1H), 2.92 (spt, *J* = 7.1 Hz, 1H), 2.79-2.87 (m, 2H), 2.66-2.75 (m, 2H), 2.35 (s, 6H), 1.29 (d, *J =* 7.1 Hz, 6H) ppm. **¹³C NMR (126 MHz, (D₃C)₂SO):** δ = 175.4 (C_{q}, 1C), 167.3 (C_{q}, 1C), 143.8 (C_{q}, 1C), 138.5 (C_{q}, 1C), 134.7 (CH, 1C), 123.4 (CH, 1C), 121.0 (CH, 1C), 119.5 (C_{q}, 1C), 111.3 (CH, 1C), 110.3 (C_{q}, 1C), 109.3 (CH, 1C), 59.5 (CH₂, 1C), 44.3 (CH₃, 2C), 33.5 (CH, 1C), 23.5 (CH₂, 1C), 18.8 (CH₃, 2C) ppm. **HR MS (ESI⁺):** m/z [M+Na]⁺ calcd. for [C₁₆H₂₂N₂NaO₂]⁺: 297.1573, found 297.1569; [M+H]⁺ calcd. for [C₁₆H₂₃N₂O₂]⁺: 275.1754, found 275.1750.

### 4-(2-Furoyl)oxy-N,N-dimethyltryptammonium fumarate (7b)

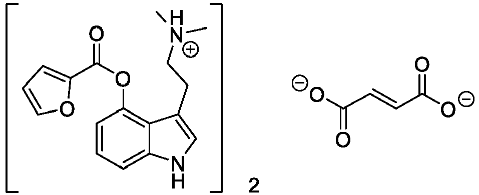

4-Hydroxy-*N*,*N*-dimethyltryptamine **1** (202.8 mg, 993 µmol, 1.00 eq) was reacted with 2-furoyl chloride (99.9 µl, 130 mg, 993 µmol, 1.00 eq) for 3 h, purified by HPLC and subsequently converted into 4-(2-furoyl)oxy-*N*,*N*-dimethyltryptamine (**19b**) by alkaline extraction as described in **GP1. 19b** (237 mg, 795 µmol, 80 %) was obtained as a colorless solid.

**¹H NMR (300 MHz, (D₃C)₂CO):** δ = 10.22 (br s, 1H), 7.95 (dd, *J* = 1.6 Hz, *J'* = 0.7 Hz, 1H), 7.57 (dd, *J* = 3.5 Hz, *J'* = 0.7 Hz, 1H), 7.32 (d, *J* = 7.8 Hz, 1H), 7.18 (s, 1H), 7.11 (t, *J* = 7.9 Hz, 1H), 6.86 (d, *J* = 7.6 Hz, 1H), 6.77 (dd, *J* = 3.5 Hz, *J'* = 1.8 Hz, 1H), 2.83-2.92 (m, 2H), 2.41-2.59 (m, 2H), 2.08 (s, 6H) ppm. **¹³C NMR (75 MHz, (D₃C)₂CO):** δ = 157.8 (C_{q}, 1C), 148.6 (CH, 1C), 145.3 (C_{q}, 1C), 144.5 (C_{q}, 1C), 140.0 (C_{q}, 1C), 124.2 (CH, 1C), 122.0 (CH, 1C), 121.1 (C_{q}, 1C), 120.2 (CH, 1C), 113.3 (C_{q}, 1C), 113.2 (CH, 1C), 112.7 (CH, 1C), 110.4 (CH, 1C), 61.9 (CH₂, 1C), 45.5 (CH₃, 2C), 25.6 (CH₂, 1C) ppm. **HR MS (ESI⁺):** m/z [M+Na]⁺ calcd. for [C₁₇H₁₈N₂NaO₃]⁺: 321.1210, found 321.1214; [M+H]⁺ calcd. for [C₁₇H₁₉N₂O₃]⁺: 299.1390, found 299.1396.

Reaction of **19b** (237 mg, 795 µmol, 1.00 eq) with fumaric acid according to GP1 yielded **7b** (281.2 mg, 789 µmol, 99 %) as a colorless solid.

**¹H NMR (500 MHz, (D₃C)₂SO):** δ = 11.17 (br s, 1H), 8.12 (d, J = 0.9 Hz, 1H), 7.65 (dd, *J* = 3.4 Hz, *J'* = 0.6 Hz, 1H), 7.30 (d, *J* = 7.7 Hz, 1H), 7.20 (d, *J* = 2.1 Hz, 1H), 7.09 (t, *J* = 7.9 Hz, 1H), 6.78-6.88 (m, 2H), 6.51 (s, 1H), 2.76-2.86 (m, 2H), 2.60-2.68 (m, 2H), 2.18 (s, 6H) ppm. **¹³C NMR (126 MHz, (D₃C)₂SO):** δ = 167.4 (C_{q}, 1C), 156.8 (C_{q}, 1C), 148.6 (CH, 1C), 143.3 (C_{q}, 1C), 142.8 (C_{q}, 1C), 138.6 (C_{q}, 1C), 134.8 (CH, 1C), 124.0 (CH, 1C), 121.1 (CH, 1C), 120.2 (CH, 1C), 119.5 (C_{q}, 1C), 112.8 (CH, 1C), 111.6 (CH, 1C), 110.2 (C_{q}, 1C), 109.8 (CH, 1C), 59.6 (CH₂, 1C), 43.9 (CH₃, 2C), 23.1 (CH₂, 1C) ppm. **HR MS (ESI⁺):** m/z [M+Na]⁺ calcd. for [C₁₇H₁₈N₂NaO₃]⁺: 321.1210, found 321.1206; [M+H]⁺ calcd. for [C₁₇H₁₉N₂O₃]⁺: 299.1390, found 299.1391.

### 4-(Cyclopropanecarbonyloxy)-N,N-dimethyltryptammonium fumarate (7c)

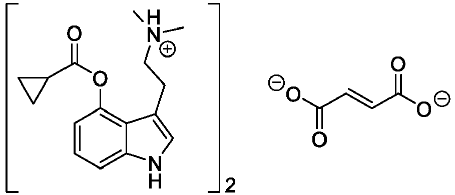

4-Hydroxy-*N*,*N*-dimethyltryptamine **1** (198 mg, 970 µmol, 1.00 eq) was reacted with cyclopropanecarboxylic acid chloride (89 µL, 101 mg, 970 µmol, 1.00 eq) for 3.5 h and evaporated to dryness. Due to incomplete consumption, the crude was again dissolved in anhydrous THF (2.8 mL) and reacted with cyclopropanecarboxylic acid chloride (24 µL, 25.4 mg, 243 µmol, 0.25 eq) and triethylamine (34 µL, 24.6 mg, 243 µmol, 0.25 eq) for 1.5 h at ambient temperature. The crude was purified by HPLC and subsequently converted into 4-(cyclopropanecarbonyloxy)-*N*,*N*-dimethyltryptamine (**19c**) by alkaline extraction as described in **GP1. 19c** (232.8 mg, 855 µmol, 88 %) was obtained as a colorless solid with a melting point of 106-109 °C. In contrast to the free bases of the other prodrugs described in this application, the free base of compound **7c** is solid at room temperature, which may be useful with respect to formulation as API in mucoadhesive patches, lozenges, sublingual tables, or other non-classical dosage forms that circumvent the gastrointestinal passage.

**¹H NMR (500 MHz, (CD₃)₂CO):** δ = 10.15 (br s, 1H), 7.25 (d, *J* = 8.1 Hz, 1H), 7.15 (s, 1H), 7.04 (t, *J* = 7.9 Hz, 1H), 6.70 (dd, *J* = 7.7 Hz, *J'* = 0.7 Hz, 1H), 2.87-2.95 (m, 2H), 2.50-2.59 (m, 2H), 2.25 (s, 6H), 1.96-2.03 (m, 1H), 1.03-1.14 (m, 4H) ppm. **¹³C NMR (126 MHz, CD₃CN):** δ = 175.0 (C_{q}, 1C), 145.2 (C_{q}, 1C), 139.7 (C_{q}, 1C), 124.2 (CH, 1C), 122.3 (CH, 1C), 121.0 (C_{q}, 1C), 113.2 (C_{q}, 1C), 112.8 (CH, 1C), 110.3 (CH, 1C), 61.6 (CH₂, 1C), 45.5 (CH₃, 2C), 25.2 (CH₂, 1C), 13.5 (CH, 1C), 9.5 (CH₂, 2C) ppm. **HR MS (ESI⁺):** m/z [M+Na]⁺ calcd. for [C₁₆H₂₀N₂NaO₂]⁺: 295.1417, found 295.1427.

Reaction of **19c** (219 mg, 805 µmol, 1.00 eq) with fumaric acid according to **GP1** yielded **7c** (257 mg, 777 µmol, 97 %) as a colorless powder.

**¹H NMR (500 MHz, (CD₃)₂SO):** δ = 11.05 (br s, 1H), 7.21 (d, *J* = 8.1 Hz, 1H), 7.14 (d, *J* = 1.3 Hz, 1H), 7.01 (t, *J* = 7.9 Hz, 1H), 6.63 (d, *J* = 7.6 Hz, 1H), 6.52 (s, 1H), 2.79-2.87 (m, 2H), 2.63-2.73 (m, 2H), 2.34 (s, 6H), 1.94-2.02 (m, 1H), 0.96-1.10 (m, 4H) ppm. **¹³C NMR (126 MHz, (CD₃)₂SO):** δ = 173.4 (C_{q}, 1C), 166.9 (C_{q}, 1C), 143.6 (C_{q}, 1C), 138.5 (C_{q}, 1C), 134.5 (CH, 1C), 123.6 (CH, 1C), 121.0 (CH, 1C), 119.6 (C_{q}, 1C), 111.4 (CH, 1C), 110.4 (C_{q}, 1C), 109.4 (CH, 1C), 59.8 (CH₂, 1C), 44.3 (CH₃, 2C), 23.3 (CH₂, 1C), 12.6 (CH, 1C), 8.9 (CH₂, 2C) ppm. **HR MS (ESI⁺):** m/z [M+Na]⁺ calcd. for [C₁₆H₂₀N₂NaO₂]⁺: 295.1417, found 295.1412; [M+H]⁺ calcd. for [C₁₆H₂₁N₂O₂]⁺: 273.1598, found 273.1596.

### Gram-scale synthesis of 7c:

4-Hydroxy-*N,N*-dimethyltryptamine **1** (896 mg, 4.39 mmol, 1.00 eq) was subjected to esterification as described above for the small scale. After evaporation of volatiles, the crude was partitioned between diethyl ether (290 mL) and carbonate buffer (0.1 M, pH 10.6, 140 mL), and the organic phase was washed twice with carbonate buffer (0.1 M, pH 10.6, 2x90 mL). Drying over Na₂SO₄ followed by rotary evaporation afforded **19c** (1.15 g, 4.24 mmol, 97 %) as a brownish solid. This was dissolved in acetone (12.0 mL) and added to a solution of fumaric acid (246 mg, 2.12 mmol, 0.50 eq) in acetone (49 mL). After standing at 5 °C for 1.5 h, the colorless precipitate was isolated by filtration over a Buchner funnel and washing with cold acetone (2x10 mL). Drying at 40 °C afforded **7c** (1.30 g, 3.93 mmol, 93 %) as a colorless, free-flowing solid.

### 4-Acetoxy-N-ethyl-N-methyltryptammonium fumarate (8a) - Reference Example

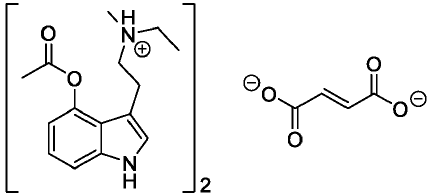

4-Acetoxy-*N*-ethyl-*N*-methyltryptamine was purchased from a commercial supplier as a brown powder and purified via RP-HPLC. The obtained trifluoroacetate salt (80.7 mg, 216 µmol, 1.00 eq) was converted into 4-acetoxy-*N*-ethyl-*N*-methyltryptamine (**20a**) using Amberlyst A-21 and subsequently reacted with fumaric acid as described in **GP1.** This yielded **8a** (33.1 mg, 104 µmol, 48 %) as a colorless solid.

### Free base 20a:

**¹H NMR (300 MHz, (CD₃)₂CO):** δ = 10.20 (br s, 1H), 7.25 (d, *J* = 8.2 Hz, 1H), 7.14 (s, 1H), 7.05 (t, *J* = 7.9 Hz, 1H), 6.72 (d, *J* = 7.6 Hz, 1H), 2.82-2.97 (m, 2H), 2.55-2.65 (m, 2H), 2.45 (q, *J* = 7.1 Hz, 2H), 2.37 (s, 3H), 2.26 (s, 3H), 1.05 (t, *J* = 7.2 Hz, 3H) ppm.

### Fumarate salt 8a:

**¹H NMR (300 MHz, (CD₃)₂SO):** δ = 11.10 (br s, 1H), 7.24 (d, *J =* 8.1 Hz, 1H), 7.18 (d, *J* = 1.9 Hz, 1H), 7.04 (t, *J* = 7.8 Hz, 1H), 6.68 (d, *J* = 7.6 Hz, 1H), 6.52 (s, 1H), 2.83-2.92 (m, 2H), 2.73-2.82 (m, 2H), 2.68 (q, *J* = 7.1 Hz, 2H), 2.41 (s, 3H), 2.36 (s, 3H), 1.08 (t, *J* = 7.2 Hz, 3H) ppm. **¹³C NMR (75 MHz, (CD₃)₂SO):** δ = 169.6 (C_{q}, 1C), 167.2 (C_{q}, 1C), 143.5 (C_{q}, 1C), 138.5 (C_{q}, 1C), 134.7 (CH, 1C), 123.8 (CH, 1C), 121.0 (CH, 1C), 119.4 (C_{q}, 1C), 111.5 (CH, 1C), 110.1 (C_{q}, 1C), 109.4 (CH, 1C), 57.3 (CH₂, 1C), 50.6 (CH₂, 1C), 40.2 (CH₃, 1C), 22.8 (CH₂, 1C), 20.9 (CH₂, 1C), 10.9 (CH₃, 1C) ppm. **HR MS (ESI⁺):** m/z [M+H]⁺ calcd. for [C₁₅H₂₁N₂O₂]⁺: 261.1598, found 261.1597. The spectroscopic data correspond to the ones previously reported in the literature.^{[30]}

### 4-Butyryloxy-N-ethyl-N-methyltryptammonium fumarate (8b)

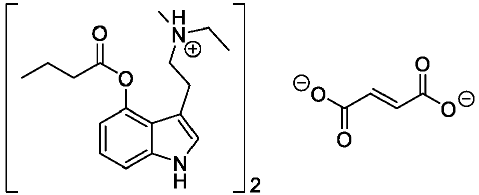

4-Hydroxy-*N*-ethyl-*N*-methyltryptamine **6** (100.0 mg, 458 µmol, 1.00 eq) was reacted with *n*-butanoyl chloride (47.6 µl, 48.8 mg, 458 µmol, 1.00 eq) for 1 h, purified by HPLC, converted into 4-butyryloxy-*N*-ethyl-*N*-methyltryptamine (**20b**) using Amberlyst A-21 and finally reacted with fumaric acid according to **GP1.** This yielded the pharmaceutically acceptable fumarate salt **8b** (38.4 mg, 111 µmol, 24 %) as a colorless solid.

### Free base 20b:

**¹H NMR (300 MHz, (CD₃)₂CO):** δ = 10.17 (br s, 1H), 7.25 (d, *J* = 8.2 Hz, 1H), 7.14 (s, 1H), 7.05 (t, *J* = 7.9 Hz, 1H), 6.71 (d, *J* = 7.8 Hz, 1H), 2.82-2.95 (m, 2H), 2.70 (t, *J* = 7.3 Hz, 2H), 2.53-2.65 (m, 2H), 2.45 (q, *J* = 7.1 Hz, 2H), 2.26 (s, 3H), 1.80 (sxt, *J* = 7.4 Hz, 2H), 1.06 (m, 6H) ppm.

### Fumarate salt 8b:

**¹H NMR (500 MHz, (CD₃)₂SO):** δ = 11.06 (br s, 1H), 7.23 (d, *J* = 8.1 Hz, 1H), 7.16 (d, *J* = 2.1 Hz, 1H), 7.03 (t, *J* = 7.8 Hz, 1H), 6.65 (dd, *J* = 7.6 Hz, *J'* = 0.7 Hz, 1H), 6.52 (s, 1H), 2.62-2.87 (m, 6H), 2.56 (q, *J* = 7.0 Hz, 2H), 2.32 (s, 3H), 1.70 (sxt, *J* = 7.4 Hz, 2H), 1.04 (t, *J* = 7.2 Hz, 3H), 1.00 (t, *J* = 7.5 Hz, 3H) ppm. **¹³C NMR (126 MHz, CD₃OD):** δ = 174.3 (C_{q}, 1C), 174.2 (C_{q}, 1C), 145.1 (C_{q}, 1C), 140.6 (C_{q}, 1C), 137.1 (CH, 1C), 125.2 (CH, 1C), 122.9 (CH, 1C), 120.5 (C_{q}, 1C), 113.1 (CH, 1C), 110.6 (CH, 1C), 109.3 (C_{q}, 1C), 57.6 (CH₂, 1C), 52.4 (CH₂, 1C), 40.3 (CH₃, 1C), 37.0 (CH₂, 1C), 22.9 (CH₂, 1C), 19.4 (CH₂, 1C), 14.0 (CH₃, 1C), 9.8 (CH₃, 1C) ppm. **HR MS (ESI⁺):** m/z [M+H]⁺ calcd. for [C₁₇H₂₅N₂O₂]⁺: 289.1911, found 289.1911.

### 4-Hexanoyloxy-N-ethyl-N-methyltryptammonium fumarate (8c) - Reference Example

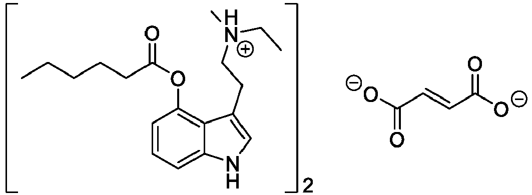

4-Hydroxy-*N*-ethyl-*N*-methyltryptamine **6** (100 mg, 458 µmol, 1.00 eq) was reacted with n-hexanoyl chloride (65.3 µl, 61.7 mg, 458 µmol, 1.00 eq) for 1.75 h, purified by HPLC, converted into 4-hexanoyloxy-*N*-ethyl-*N*-methyltryptamine (**20c**) using Amberlyst A-21 and finally reacted with fumaric acid according to **GP1.** This yielded the pharmaceutically acceptable fumarate salt **8c** (83.7 mg, 224 µmol, 49 %) as a colorless solid.

### Free base 20c:

**¹H NMR (300 MHz, (CD₃)₂CO):** δ = 10.18 (br s, 1H), 7.25 (d, *J* = 8.2 Hz, 1H), 7.13 (s, 1H), 7.05 (t, *J* = 7.9 Hz, 1H), 6.71 (d, *J* = 7.8 Hz, 1H), 2.82-2.95 (m, 2H), 2.72 (t, *J* = 7.5 Hz, 2H), 2.55-2.65 (m, 2H), 2.45 (q, *J* = 7.2 Hz, 2H), 2.26 (s, 3H), 1.78 (quin, *J* = 7.3 Hz, 2H), 1.33-1.53 (m, 4H), 1.05 (t, *J* = 7.1 Hz, 3H), 0.95 (t, *J* = 6.9 Hz, 3H) ppm.

### Fumarate salt 8c:

**¹H NMR (500 MHz, (CD₃)₂SO):** δ = 11.08 (br s, 1H), 7.23 (dd, *J* = 8.1 Hz, *J*' = 0.6 Hz, 1H), 7.17 (d, *J* = 2.3 Hz, 1H), 7.03 (t, *J* = 7.9 Hz, 1H), 6.65 (dd, *J* = 7.6 Hz, *J*' = 0.6 Hz, 1H), 6.51 (s, 1H), 2.81-2.89 (m, 2H), 2.71-2.77 (m, 2H), 2.68 (t, *J* = 7.5 Hz, 2H), 2.62 (q, *J* = 7.2 Hz, 2H), 2.36 (s, 3H), 1.68 (quin, *J* = 7.4 Hz, 2H), 1.29-1.41 (m, 4H), 1.06 (t, *J* = 7.2 Hz, 3H), 0.90 (t, *J* = 7.0 Hz, 3H) ppm. **¹³C NMR (126 MHz, (CD₃)₂SO):** δ = 172.2 (C_{q}, 1C), 167.4 (C_{q}, 1C), 143.6 (C_{q}, 1C), 138.5 (C_{q}, 1C), 134.8 (CH, 1C), 123.6 (CH, 1C), 121.0 (CH, 1C), 119.5 (C_{q}, 1C), 111.5 (CH, 1C), 110.3 (C_{q}, 1C), 109.4 (CH, 1C), 57.4 (CH₂, 1C), 50.6 (CH₂, 1C), 40.3 (CH₃, 1C), 33.4 (CH₂, 1C), 30.7 (CH₂, 1C), 24.0 (CH₂, 1C), 23.1 (CH₂, 1C), 21.8 (CH₂, 1C), 13.8 (CH₃, 1C), 11.1 (CH₃, 1C) ppm. **HR MS (ESI⁺):** m/z [M+H]⁺ calcd. for [C₁₉H₂₉N₂O₂]⁺: 317.2224 , found 317.2228.

### 4-iso-Butyryloxy-N-ethyl-N-methyltryptammonium fumarate (8d)

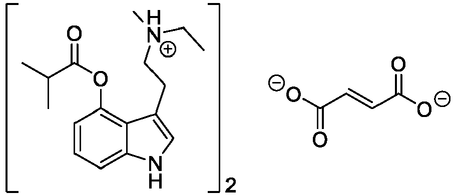

4-Hydroxy-*N*-ethyl-*N*-methyltryptamine **6** (100.0 mg, 458 µmol, 1.00 eq) was reacted with iso-butyryl chloride (48.0 µl, 48.8 mg, 458 µmol, 1.00 eq), purified by HPLC, converted into 4-iso-butyryloxy-*N*-ethyl-*N*-methyltryptamine (**20d**) using Amberlyst A-21 and finally reacted with fumaric acid according to **GP1.** This yielded the pharmaceutically acceptable fumarate salt **8d** (41.2 mg, 119 µmol, 26 %) as an off-whitesolid.

### Free base 20d:

**¹H NMR (300 MHz, (CD₃)₂CO):** δ = 10.17 (br s, 1H), 7.25 (d, *J* = 8.2 Hz, 1H), 7.14 (s, 1H), 7.05 (t, *J* = 7.9 Hz, 1H), 6.69 (d, *J* = 7.6 Hz, 1H), 2.84-3.03 (m, 3H), 2.55-2.67 (m, 2H), 2.45 (q, *J* = 7.1 Hz, 2H), 2.25 (s, 3H), 1.36 (d, *J* = 6.9 Hz, 6H), 1.04 (t, *J* = 7.1 Hz, 3H) ppm.

### Fumarate salt 8d:

**¹H NMR (500 MHz, CD₃OD):** δ = 7.25-7.31 (m, 1H), 7.23 (s, 1H), 7.12 (t, *J* = 7.9 Hz, 1H), 6.71 (dd, *J* = 7.7 Hz, J` = 0.6 Hz, 1H), 6.68 (s, 1H), 3.44 (t, *J =* 7.3 Hz, 2H), 3.14-3.26 (m, 4H), 2.96 (dt, *J* = 13.9 Hz, *J'* = 7.0 Hz, 1H), 2.84 (s, 3H), 1.38 (d, *J* = 7.0 Hz, 6H), 1.28 (t, *J* = 7.3 Hz, 3H) ppm. **¹³C NMR (126 MHz, CD₃OD):** δ = 177.8 (C_{q}, 1C), 172.1 (C_{q}, 1C), 145.4 (C_{q}, 1C), 140.7 (C_{q}, 1C), 136.4 (CH, 1C), 125.3 (CH, 1C), 123.1 (CH, 1C), 120.5 (C_{q}, 1C), 113.0 (CH, 1C), 110.6 (CH, 1C), 108.7 (C_{q}, 1C), 57.3 (CH₂, 1C), 52.6 (CH₂, 1C), 40.3 (CH₃, 1C), 35.4 (CH, 1C), 22.8 (CH₂, 1C), 19.5 (CH₃, 2C), 9.5 (CH₃, 1C) ppm. **HR MS (ESI⁺):** m/z [M+H]⁺ calcd. for [C₁₇H₂₅N₂O₂]⁺: 289.1911, found 289.1911.

### 4-(Cyclopropanecarbonyloxy)-N-ethyl-N-methyltryptammonium fumarate (8e)

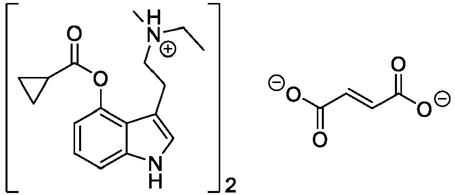

4-Hydroxy-*N*-ethyl-*N*-methyltryptamine **6** (100.0 mg, 458 µmol, 1.00 eq) was reacted with cyclopropanecarboxylic acid chloride (41.6 µl, 47.9 mg, 458 µmol, 1.00 eq), purified by HPLC, converted into 4-(cyclopropanecarbonyloxy)-*N*-ethyl-*N*-methyltryptamine (**20e**) by alkaline extraction and finally reacted with fumaric acid according to **GP1.** This yielded the pharmaceutically acceptable fumarate salt **8e** (121.4 mg, 352 µmol, 77 %) as a colorless solid.

### Free base 20e:

**¹H NMR (500 MHz, (D₃C)₂CO):** δ = 10.15 (br s, 1H), 7.25 (d, *J* = 8.1 Hz, 1H), 7.15 (s, 1H), 7.04 (t, *J* = 7.9 Hz, 1H), 6.70 (d, *J* = 7.7 Hz, 1H), 2.88-2.98 (m, 2H), 2.59-2.70 (m, 2H), 2.46 (q, *J* = 7.1 Hz, 2H), 2.27 (s, 3H), 1.96-2.03 (m, 1H), 1.06-1.15 (m, 4H), 1.04 (t, *J* = 7.2 Hz, 3H) ppm.

### Fumarate salt 8e:

**¹H NMR (500 MHz, (D₃C)₂SO):** δ = 11.09 (br s, 1H), 7.23 (dd, *J* = 8.1 Hz, *J'* = 0.6 Hz, 1H), 7.17 (d, *J* = 2.2 Hz, 1H), 7.03 (t, *J* = 7.8 Hz, 1H), 6.65 (d, *J* = 7.6 Hz, 1H), 6.51 (s, 1H), 2.83-2.91 (m, 2H), 2.73-2.81 (m, 2H), 2.63 (q, *J* = 7.2 Hz, 2H), 2.38 (s, 3H), 1.94-2.06 (m, 1H), 0.99-1.10 (m, 7H) ppm. **¹³C NMR (126 MHz, (D₃C)₂SO):** δ = 173.4 (C_{q}, 1C), 167.3 (C_{q}, 1C), 143.6 (C_{q}, 1C), 138.5 (C_{q}, 1C), 134.8 (CH, 1C), 123.6 (CH, 1C), 121.0 (CH, 1C), 119.6 (C_{q}, 1C), 111.4 (CH, 1C), 110.4 (C_{q}, 1C), 109.4 (CH, 1C), 57.3 (CH₂, 1C), 50.5 (CH₂, 1C), 40.3 (CH₃, 1C), 22.8 (CH₂, 1C), 12.6 (CH, 1C), 11.2 (CH₃, 1C), 8.9 (CH₂, 2C) ppm. **HR MS (ESI⁺):** m/z [M+Na]⁺ calcd. for [C₁₇H₂₂N₂NaO₂]⁺: 309.1573, found 309.1567; [M+H]⁺ calcd. for [C₁₇H₂₃N₂O₂]⁺: 287.1754, found 287.1747.

### 4-Pivaloyloxy-N-ethyl-N-methyltryptammonium fumarate (8f)

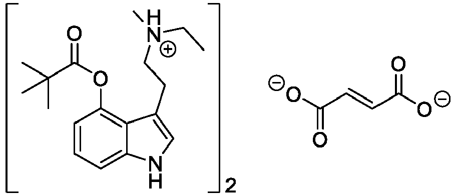

4-Hydroxy-*N*-ethyl-*N*-methyltryptamine **6** (100 mg, 458 µmol, 1.00 eq) was reacted with pivaloyl chloride (980 µl, 959 mg, 7.96 mmol, 17.4 eq) for 1 h, purified by HPLC, converted into 4-pivaloyloxy-*N*-ethyl-*N*-methyltryptamine (**20f**) using Amberlyst A-21 and finally reacted with fumaric acid according to **GP1.** This yielded the pharmaceutically acceptable fumarate salt **8f** (56.1 mg, 156 µmol, 34 %) as a colorless solid. Free base **20f:**

**¹H NMR (300 MHz, (CD₃)₂CO):** δ = 10.18 (br s, 1H), 7.25 (d, *J* = 8.2 Hz, 1H), 7.14 (s, 1H), 7.05 (t, *J* = 7.8 Hz, 1H), 6.62 (d, *J* = 7.6 Hz, 1H), 2.85-2.98 (m, 2H), 2.57-2.70 (m, 2H), 2.44 (q, *J* = 7.2 Hz, 2H), 2.24 (s, 3H), 1.44 (s, 9H), 1.03 (t, *J* = 7.1 Hz, 3H) ppm.

### Fumarate salt 8f:

**¹H NMR (500 MHz, CD₃OD):** δ = 7.29 (dd, *J* = 8.2 Hz, *J*' = 0.6 Hz, 1H), 7.25 (s, 1H), 7.13 (t, *J* = 7.9 Hz, 1H), 6.69 (s, 1H), 6.64 (dd, *J* = 7.7 Hz, *J*' = 0.6 Hz, 1H), 3.46-3.52 (m, 2H), 3.20 (s, 4H), 2.84 (s, 3H), 1.45 (s, 9H), 1.27 (t, *J* = 7.3 Hz, 3H) ppm. **¹³C NMR (126 MHz, CD₃OD):** δ = 179.5 (C_{q}, 1C), 171.5 (C_{q}, 1C), 145.8 (C_{q}, 1C), 140.8 (C_{q}, 1C), 136.3 (CH, 1C), 125.0 (CH, 1C), 123.2 (CH, 1C), 120.7 (C_{q}, 1C), 113.0 (CH, 1C), 110.7 (CH, 1C), 108.6 (C_{q}, 1C), 57.0 (CH₂, 1C), 52.7 (CH₂, 1C), 40.4 (CH₃, 1C), 40.4 (C_{q}, 1C), 27.7 (CH₃, 3C), 22.9 (CH₂, 1C), 9.5 (CH₃, 1C) ppm. **HR MS (ESI⁺):** m/z [M+H]⁺ calcd. for [C₁₈H₂₇N₂O₂]⁺: 303.2067, found 303.2078.

### 4-(3-Methylbutanoyl)oxy-N-ethyl-N-methyltryptammonium fumarate (8g)

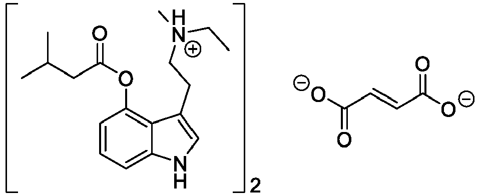

4-Hydroxy-*N*-ethyl-*N*-methyltryptamine **6** (100 mg, 458 µmol, 1.00 eq) was reacted with 3-methylbutanoyl chloride (55.9 µl, 55.2 mg, 458 µmol, 1.00 eq) for 1 h, purified by HPLC, converted into 4-(3-methylbutanoyl)oxy-*N*-ethyl-*N*-methyltryptamine (**20g**) using Amberlyst A-21 and finally reacted with fumaric acid according to **GP1.** This yielded the pharmaceutically acceptable fumarate salt **8g** (33.1 mg, 91.8 µmol, 20 %) as a colorless solid.

### Free base 20g:

**¹H NMR (300 MHz, (CD₃)₂CO):** δ = 10.19 (br s, 1H), 7.26 (d, *J* = 8.2 Hz, 1H), 7.13 (s, 1H), 7.06 (t, *J* = 7.9 Hz, 1H), 6.72 (d, *J* = 7.6 Hz, 1H), 2.82-2.96 (m, 2H), 2.54-2.67 (m, 4H), 2.46 (q, *J* = 7.0 Hz, 2H), 2.18-2.34 (m, 4H), 0.98-1.13 (m, 9H) ppm.

### Fumarate salt 8g:

**¹H NMR (500 MHz, (CD₃)₂SO):** δ = 11.10 (br s, 1H), 7.24 (d, *J* = 8.1 Hz, 1H), 7.19 (d, *J* = 2.1 Hz, 1H), 7.04 (t, *J* = 7.8 Hz, 1H), 6.65 (d, *J* = 7.6 Hz, 1H), 6.54 (s, 1H), 2.77-2.91 (m, 4H), 2.65-2.74 (m, 2H), 2.58 (d, *J* = 7.1 Hz, 2H), 2.43 (s, 3H), 2.09-2.21 (non, *J* = 6.8 Hz, 1H), 1.08 (t, *J* = 7.2 Hz, 3H), 1.03 (d, *J* = 6.6 Hz, 6H) ppm. **¹³C NMR (126 MHz, (CD₃)₂SO):** δ = 171.5 (C_{q}, 1C), 167.0 (C_{q}, 1C), 143.5 (C_{q}, 1C), 138.5 (C_{q}, 1C), 134.6 (CH, 1C), 123.7 (CH, 1C), 121.1 (CH, 1C), 119.4 (C_{q}, 1C), 111.5 (CH, 1C), 110.0 (C_{q}, 1C), 109.4 (CH, 1C), 57.2 (CH₂, 1C), 50.6 (CH₂, 1C), 42.3 (CH₂, 1C), 40.2 (CH₃, 1C), 25.1 (CH, 1C), 22.9 (CH₂, 1C), 22.2 (CH₃, 2C), 10.9 (CH₃, 1C) ppm. **HR MS (ESI⁺):** m/z [M+H]⁺ calcd. for [C₁₈H₂₇N₂O₂]⁺: 303.2067, found 303.2070.

### 4-(tert-Butylacetyl)oxy-N-ethyl-N-methyltryptammonium fumarate (8h)

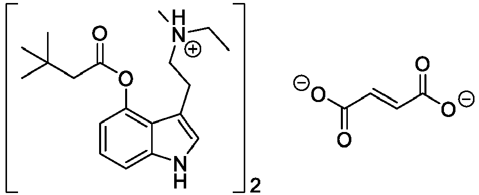

4-Hydroxy-*N*-ethyl-*N*-methyltryptamine **6** (100 mg, 458 µmol, 1.00 eq) was reacted with *tert*-butylacetyl chloride (64.9 µl, 61.7 mg, 458 µmol, 1.00 eq) for 3.5 h, purified by HPLC, converted into 4-(*tert*-butylacetyl)oxy-*N*-ethyl-*N*-methyltryptamine (**20h**) using Amberlyst A-21 and finally reacted with fumaric acid according to **GP1.** This yielded the pharmaceutically acceptable fumarate salt **8h** (111.2 mg, 297 µmol, 65 %) as a colorless solid.

### Free base 20h:

**¹H NMR (300 MHz, (CD₃)₂CO):** δ = 10.20 (br s, 1H), 7.25 (d, *J* = 8.1 Hz, 1H), 7.13 (s, 1H), 7.06 (t, *J* = 7.9 Hz, 1H), 6.73 (d, *J* = 7.5 Hz, 1H), 2.84-2.97 (m, 2H), 2.55-2.69 (m, 4H), 2.47 (q, *J* = 7.2 Hz, 2H), 2.28 (s, 3H), 1.18 (s, 9H), 1.07 (t, *J* = 7.2 Hz, 3H) ppm.

### Fumarate salt 8h:

**¹H NMR (500 MHz, (CD₃)₂SO):** δ = 11.09 (br s, 1H), 7.23 (dd, *J* = 8.1 Hz, *J'* = 0.7 Hz, 1H), 7.17 (d, *J* = 2.2 Hz, 1H), 7.03 (t, *J* = 7.8 Hz, 1H), 6.63 (dd, *J* = 7.6 Hz, *J'* = 0.7 Hz, 1H), 6.51 (s, 1H), 2.82-2.89 (m, 2H), 2.70-2.79 (m, 2H), 2.63 (q, *J* = 7.2 Hz, 2H), 2.57 (s, 2H), 2.37 (s, 3H), 1.11 (s, 9H), 1.06 (t, *J =* 7.1 Hz, 3H) ppm. **¹³C NMR (126 MHz, (CD₃)₂SO):** δ = 171.1 (C_{q}, 1C), 167.9 (C_{q}, 1C), 143.9 (C_{q}, 1C), 139.0 (C_{q}, 1C), 135.3 (CH, 1C), 124.1 (CH, 1C), 121.5 (CH, 1C), 120.0 (C_{q}, 1C), 111.9 (CH, 1C), 110.8 (C_{q}, 1C), 109.8 (CH, 1C), 57.9 (CH₂, 1C), 51.1 (CH₂, 1C), 47.0 (CH₂, 1C), 40.8 (CH₃, 1C), 30.9 (C_{q}, 1C), 29.8 (CH₃, 3C), 23.5 (CH₂, 1C), 11.6 (CH₃, 1C) ppm. **HR MS (ESI⁺):** m/z [M+H]⁺ calcd. for [C₁₉H₂₉N₂O₂]⁺: 317.2224, found 317.2225.

### 4-Benzoyloxy-N-ethyl-N-methyltryptammonium fumarate (8i)

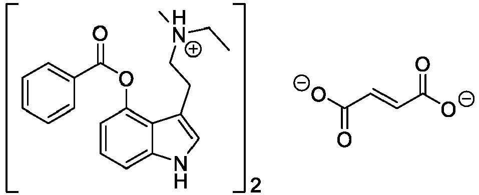

4-Hydroxy-*N*-ethyl-*N*-methyltryptamine **6** (50.0 mg, 229 µmol, 1.00 eq) was reacted with benzoyl chloride (27.7 µl, 33.8 mg, 240 µmol, 1.05 eq) for 30 min, purified by HPLC, converted into 4-benzoyloxy-*N*-ethyl-*N*-methyltryptamine (**20i**) using Amberlyst A-21 and finally reacted with fumaric acid according to **GP1.** This yielded the pharmaceutically acceptable fumarate salt **8i** (27.0 mg, 71.0 µmol, 31 %) as a colorless solid.

### Free base 20i:

**¹H NMR (300 MHz, (CD₃)₂CO):** δ = 10.29 (br s, 1H), 8.30 (d, *J* = 7.8 Hz, 2H), 7.69-7.85 (m, 1H), 7.58-7.66 (m, 2H), 7.32 (dd, *J* = 8.1 Hz, *J'* = 0.7 Hz, 1H), 7.04-7.21 (m, 2H), 6.87 (d, *J* = 7.6 Hz, 1H), 2.78-2.92 (m, 2H), 2.50-2.61 (m, 2H), 2.23 (q, *J* = 7.1 Hz, 2H), 2.00 (s, 3H), 0.85 (t, *J* = 7.1 Hz, 3H) ppm.

### Fumarate salt 8i:

**¹H NMR (500 MHz, (CD₃)₂SO):** δ = 11.13 (br s, 1H), 8.21 (dd, *J* = 8.1 Hz, *J'* = 1.0 Hz, 2H), 7.73-7.80 (m, 1H), 7.63 (t, *J* = 7.8 Hz, 2H), 7.29 (dd, *J* = 8.2 Hz, *J*' = 0.6 Hz, 1H), 7.19 (d, *J* = 2.2 Hz, 1H), 7.10 (t, *J* = 7.9 Hz, 1H), 6.81 (d, *J* = 7.6 Hz, 1H), 6.54 (s, 1H), 2.72-2.80 (m, 2H), 2.59-2.68 (m, 2H), 2.27-2.37 (m, *J* = 6.5 Hz, 2H), 2.04 (s, 3H), 0.84 ppm (t, *J* = 7.2 Hz, 3H) ppm. **¹³C NMR (126 MHz, (CD₃)₂SO):** δ = 166.7 (C_{q}, 1C), 165.1 (C_{q}, 1C), 143.6 (C_{q}, 1C), 138.6 (C_{q}, 1C), 134.4 (CH, 1C), 134.0 (CH, 1C), 129.9 (CH, 2C), 129.1 (CH, 2C), 129.0 (CH, 1C), 123.8 (CH, 1C), 121.1 (CH, 1C), 119.6 (C_{q}, 1C), 111.7 (CH, 1C), 110.3 (C_{q}, 1C), 109.7 (CH, 1C), 57.5 (CH₂, 1C), 50.4 (CH₂, 1C), 40.1 (CH₃, 1C), 23.0 (CH₂, 1C), 11.2 (CH₃, 1C) ppm. **HR MS (ESI⁺):** m/z [M+H]⁺ calcd. for [C₂₀H₂₃N₂O₂]⁺: 323.1754, found 323.1756.

### 4-(2-Furoyl)oxy-N-ethyl-N-methyltryptammonium fumarate (8j)

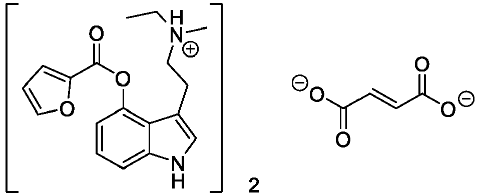

4-Hydroxy-*N*-ethyl-*N*-methyltryptamine **6** (100 mg, 458 µmol, 1.00 eq) was dissolved in anhydrous THF (0.93 mL) and reacted with 2-furoyl chloride (57.3 µl, 74.7 mg, 573 µmol, 1.25 eq) and triethylamine (96.0 µl, 69.5 mg, 687 µmol, 1.50 eq) for 1 h. After removing volatiles by rotary evaporation, the crude was purified by HPLC, converted into 4-(2-furoyl)oxy-*N*-ethyl-*N*-methyltryptamine (**20j**) by alkaline extraction and finally reacted with fumaric acid according to **GP1.** This yielded **8j** (27.7 mg, 74.8 µmol, 16 %) as a colorless solid.

### Free base 20j:

**¹H NMR (300 MHz, (D₃C)₂CO):** δ = 10.27 (br s, 1H), 7.94 (dd, *J* = 1.8 Hz, *J'* = 0.7 Hz, 1H), 7.58 (dd, *J* = 3.5 Hz, *J'* = 0.7 Hz, 1H), 7.31 (dd, *J* = 8.2 Hz, *J'* = 0.7 Hz, 1H), 7.18 (d, *J* = 2.1 Hz, 1H), 7.11 (t, *J* = 7.9 Hz, 1H), 6.87 (dd, *J* = 7.7 Hz, *J*'= 0.7 Hz, 1H), 6.76 (dd, *J* = 3.5, *J'* = 1.8 Hz, 1H), 2.81-2.93 (m, 2H), 2.51-2.67 (m, 2H), 2.30 (q, *J* = 7.2 Hz, 2H), 2.08 (s, 3H), 0.92 (t, *J* = 7.1 Hz, 3H) ppm.

### Fumarate salt 8j:

**¹H NMR (500 MHz, (D₃C)₂SO):** δ = 11.20 (br s, 1H), 8.08-8.14 (m, 1H), 7.62-7.69 (m, 1H), 7.30 (d, *J* = 8.1 Hz, 1H), 7.22 (d, *J* = 2.1 Hz, 1H), 7.09 (t, *J* = 7.9 Hz, 1H), 6.77-6.86 (m, 2H), 6.50 (s, 1H), 2.80-2.91 (m, 2H), 2.70-2.79 (m, 2H), 2.43-2.50 (m, 2H), 2.19 (s, 3H), 0.95 (t, *J* = 7.2 Hz, 3H) ppm. **¹³C NMR (126 MHz, (D₃C)₂SO):** δ = 167.6 (C_{q}, 1C), 156.8 (C_{q}, 1C), 148.6 (CH, 1C), 143.2 (C_{q}, 1C), 142.8 (C_{q}, 1C), 138.6 (C_{q}, 1C), 134.9 (CH, 1C), 124.1 (CH, 1C), 121.1 (CH, 1C), 120.2 (CH, 1C), 119.5 (C_{q}, 1C), 112.7 (CH, 1C), 111.6 (CH, 1C), 110.1 (C_{q}, 1C), 109.8 (CH, 1C), 57.2 (CH₂, 1C), 50.2 (CH₂, 1C), 39.6 (CH₃, 1C), 22.5 (CH₂, 1C), 10.9 (CH₃, 1C) ppm. The *N*-methyl ¹³C signal is superimposed by that of DMSO-ds as evidenced by the HSQC and HMBC spectra. **HR MS (ESI⁺):** m/z [M+H]⁺ calcd. for [C₁₈H₂₁N₂O₃]⁺: 313.1547, found 313.1544.

### Synthesis of acyloxymethyl prodrugs having formula (2)

### Synthesis of N-benzyloxycarbonyl derivatives

### Benzyl imidazole-1-carboxylate (9)

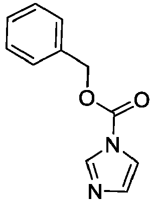

This compound was prepared following a literature procedure (cf. Heller et al. Org. Lett. 2010, 12, 4572). To an ice-cold solution of imidazole (3.76 g, 55.0 mmol, 1.00 eq) in dry THF (50 mL) was added benzyl chloroformate (4.25 mL, 5.08 g, 28.3 mmol, 0.51 eq) dropwise. The obtained suspension was stirred at 0°C for 1 h, followed by stirring for 4 h at ambient temperature. The suspended solids were then separated by filtration, and the filter cake was washed with diethyl ether (50 mL). The filtrate was concentrated under reduced pressure. Subsequently, the colorless residue was dissolved in diethyl ether (100 mL), washed with water (2x50 mL) and dried over MgSO₄. The solvent was removed under reduced pressure, which gave **9** as a colorless oil (4.59 g, 22.7 mmol, 83 %). The isolated material was found to be stable for at least 16 weeks when stored at -20°C.

**¹H NMR (300 MHz, CDCl₃):** δ = 8.15 (s, 1H), 7.34-7.49 (m, 6H), 7.01-7.12 (m, 1H), 5.42 (s, 2H) ppm. **¹³C NMR (75 MHz, CDCl₃):** δ = 148.8 (C_{q}, 1C), 137.3 (CH, 1C), 134.1 (C_{q}, 1C), 130.9 (CH, 1C), 129.3 (CH, 1C), 129.0 (CH, 2C), 128.9 (CH, 2C), 117.3 (CH, 1C), 70.0 (CH₂, 1C) ppm. **HR MS (ESI⁺):** m/z [M+Na]⁺ calcd. for [C₁₁H₁₀N₂NaO₂]⁺: 225.0634, found 225.0634. The analytical data correspond to the ones previously described in the literature.^{[32]}

### 1-Benzyloxycarbonyl-4-hydroxy-N,N-dimethyltryptamine (10)

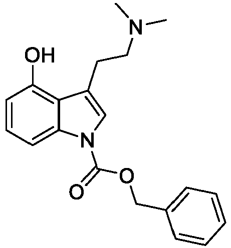

A flame-dried Schlenk flask was charged with 4-hydroxy-*N*,*N*-dimethyltryptamine **1** (1.36 g, 6.66 mmol, 1.00 eq). Benzyl 1*H*-imidazole-1-carboxylate **9** (1.48 g, 7.32 mmol, 1.10 eq) dissolved in anhydrous acetonitrile (20 mL) was added, followed by 1,8-di-azabicyclo[5.4.0]undec-7-ene (497 µl, 507 mg, 3.33 mmol, 0.50 eq). The resulting brown solution was stirred for 26 h at ambient temperature. Afterwards, the reaction mixture was concentrated by rotary evaporation, quenched by addition of 0.5 M aqueous NaOH (35 mL) and the mixture was extracted with dichloromethane (3x200 mL). The pooled organic layers were dried over Na₂SO₄ and concentrated *in vacuo.* Purification by column chromatography (ethyl acetate (EtOAc)/triethylamine (TEA) 99/1 (v/v), SiO₂ 50 g) afforded **10** (1.35 g, 3.99 mmol, 60 %) as a yellowish oil which crystallized upon standing.

**¹H NMR (500 MHz, CDCl₃):** δ = 13.45 (br s, 1H), 7.73 (br d, *J* = 5.1 Hz, 1H), 7.44-7.52 (m, 2H), 7.33-7.44 (m, 3H), 7.28 (s, 1H), 7.19 (t, *J* = 8.1 Hz, 1H), 6.74 (dd, *J* = 7.9 Hz, *J'* = 0.6 Hz, 1H), 5.42 (s, 2H), 2.84-2.92 (m, 2H), 2.66-2.74 (m, 2H), 2.37 (s, 6H) ppm. **¹³C NMR (126 MHz, CDCl₃):** δ = 152.2 (C_{q}, 1C), 150.9 (C_{q}, 1C), 137.9 (C_{q}, 1C), 135.4 (C_{q}, 1C), 128.8 (CH, 2C), 128.7 (CH, 1C), 128.5 (CH, 2C), 126.2 (CH,1C), 121.8 (CH,1C), 120.0 (C_{q}, 1C), 119.7 (C_{q}, 1C), 111.4 (CH, 1C), 106.6 (CH, 1C), 68.5 (CH₂, 1C), 61.0 (CH₂, 1C), 45.4 (CH₃, 2C) , 25.2 (CH₂, 1C) ppm. **HR MS (ESI⁺):** m/z [M+Na]⁺ calcd. for [C₂₀H₂₂N₂NaO₃]⁺: 361.1523, found 361.1512; [M+H]⁺ calcd. for [C₂₀H₂₃N₂O₃]⁺: 339.1703, found 339.1701.

### 1-Benzyloxycarbonyl-4-hydroxy-N-ethyl-N-methyltryptamine (11)

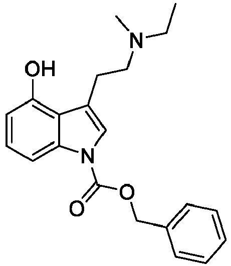

4-Hydroxy-*N*-ethyl-*N*-methyltryptamine **6** (750 mg, 3.44 mmol, 1.00 eq) was dissolved in anhydrous acetonitrile (10.3 mL) under a nitrogen atmosphere. After adding benzyl 1*H*-imidazole-1-carboxylate **9** as a frozen solid (764 mg, 3.78 mmol, 1.10 eq) followed by 1,8-diazabicyclo[5.4.0]undec-7-ene (256 µL, 262 mg, 1.72 mmol, 0.50 eq), the resulting violet solution was stirred at ambient temperature for 18 h. The reaction mixture was then concentrated under reduced pressure, mixed with a 0.5 M aqueous NaOH solution (17 mL), and extracted with dichloromethane (3x100 mL). The organic phase was dried over Na₂SO₄, concentrated and purified by column chromatography (Cyclohexane/EtOAc 100/0 -0/100 (v/v), 1 % TEA, SiO₂ 25 g), which yielded **11** (1.08 g, 3.07 mmol, 89 %) as a colorless solid.

**¹H NMR (500 MHz, CDCl₃):** δ = 13.55 (br s, 1H), 7.70 (s, 1H), 7.46-7.51 (m, 2H), 7.34-7.44 (m, 3H), 7.28 (s, 1H), 7.18 (t, *J* = 8.1 Hz, 1H), 6.72 (dd, *J* = 8.0 Hz, *J'* = 0.5 Hz, 1H), 5.42 (s, 2H), 2.87-2.93 (m, 2H), 2.71-2.76 (m, 2H), 2.55 (q, *J* = 7.1 Hz, 2H), 2.36 (s, 3H), 1.04 (t, *J* = 7.2 Hz, 3H) ppm. **¹³C NMR (126 MHz, CDCl₃):** δ = 152.2 (C_{q}, 1C), 150.9 (C_{q}, 1C), 137.9 (C_{q}, 1C), 135.4 (C_{q}, 1C), 128.8 (CH, 2C), 128.7 (CH, 1C), 128.6 (CH, 2C), 126.3 (CH, 1C), 121.8 (CH, 1C), 120.2 (C_{q}, 1C), 120.0 (C_{q}, 1C), 111.6 (CH, 1C), 106.7 (CH, 1C), 68.6 (CH₂, 1C), 58.7 (CH₂, 1C), 52.2 (CH₂, 1C), 42.3 (CH₃, 1C), 25.4 (CH₂, 1C), 11.4 (CH₃, 1C) ppm. **HR MS (ESI⁺):** m/z [M+H]⁺ calcd. for [C₂₁H₂₅N₂O₃]⁺: 353.1860, found 353.1861.

### Synthesis of iodomethyl carboxylates

### General procedure (GP2): iodination of chloromethyl carboxylates

This protocol is based on a literature procedure (cf. Bandgar et al. J. Med. Chem. 2011, 54, 1191; and Jones et al. Beilstein J. Org. Chem. 2019, 15, 801). A 3.3 M solution of chloromethyl carboxylate (1.00 eq) in anhydrous acetonitrile was allowed to react with sodium iodide (1.80 eq) at 30-35 °C under a nitrogen atmosphere in a flame-dried Schlenk tube under exclusion of light. Subsequently, dichloromethane (1.4 mL per mmol of chloromethyl carboxylate) and water (1.4 mL per mmol of chloromethyl carboxylate) were added, and the biphasic mixture was stirred for 10 min. The organic phase was separated, washed with 2 % aqueous Na₂S₂O₃ solution (0.7 mL per mmol of chloromethyl carboxylate) and dried over Na₂SO₄. Evaporation *in vacuo* at ambient temperature afforded the target product in good to excellent yield. Iodomethyl carboxylates were stored at 2-8°C under exclusion of light and under inert gas.

### lodomethyl pivalate (12a)

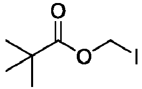

Chloromethyl pivalate (957 µL, 1.00 g, 6.64 mmol, 1.00 eq) was subjected to the reaction conditions outlined in **GP2** for 16 h. Aqueous workup followed by evaporation at ambient temperature afforded **12a** (1.17 g, 4.83 mmol, 73 %) as a yellowish oil. **¹H NMR (300 MHz, CDCl₃):** δ =5.92 (s, 2H), 1.19 (s, 9H) ppm. **¹³C NMR (75 MHz, CDCl₃):** δ = 176.5 (C_{q}, 1C), 39.0 (C_{q}, 1C), 31.5 (CH₂, 1C), 26.7 (CH₃, 3C) ppm. In line with the literature, the molecular ion was not detectable by ESI⁺ MS of a solution of **12a** in acetonitrile.^{[19a]} The spectroscopic properties correspond to the ones previously reported in the literature.^{[18-19]}

### lodomethyl butyrate (12b)

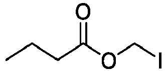

Chloromethyl butyrate (1.90 mL, 2.00 g, 14.6 mmol, 1.00 eq) was subjected to the reaction conditions outlined in **GP2** for 6 h. Aqueous workup followed by evaporation at ambient temperature yielded **12b** (3.02 g, 13.2 mmol, 90 %) as a colorless to slightly yellowish oil.

**¹H NMR (300 MHz, CDCl₃):** δ = 5.91 (s, 2H), 2.31 (t, *J* = 7.3 Hz, 2H), 1.68 (sxt, *J* = 7.4 Hz, 2H), 0.96 (t, *J* = 7.4 Hz, 3H) ppm. **¹³C NMR (75 MHz, CDCl₃):** δ = 171.8 (C_{q}, 1C), 36.3 (CH₂, 1C), 30.7 (CH₂, 1C), 18.2 (CH₂, 1C), 13.7 (CH₃, 1C) ppm. The molecular ion was not detectable by ESI⁺ MS of a solution of **12b** in acetonitrile.

### Synthesis of acyloxymethyl derivatives of 4-hydroxytryptamines

### 1-Benzyloxycarbonyl-4-pivaloyloxymethyloxy-N,N-dimethyltryptammonium trifluoroacetate (13a)

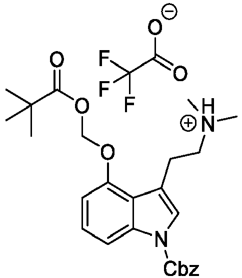

A solution of **10** (550 mg, 1.63 mmol, 1.00 eq) in anhydrous DMF (7.6 mL) was treated with NaH (60 % suspension in mineral oil, 97.5 mg, 2.44 mmol, 1.50 eq) at 0 °C. After 25 min at 0 °C, gas generation ceased and a clear brown solution was obtained. The temperature was then lowered to -30 to -40 °C. Iodomethyl pivalate **12a** (413 mg, 1.71 mmol, 1.05 eq) as a solution in anhydrous THF (7.6 mL) was added dropwise. The reaction mixture was allowed to warm to ambient temperature, and stirring was continued for 5 h. Then, the reaction mixture was poured into aq. 1 N NaOH (7.7 mL) and extracted with ethyl acetate (3x60 mL). The organic layer was dried over Na₂SO₄, concentrated, dissolved in acetonitrile (+0.1 % TFA) and purified by means of reversed-phase chromatography on a preparative HPLC instrument (conditions I). Evaporation of volatiles followed by lyophilization afforded **13a** (335 mg, 591 µmol, 36 %) as a yellowish oil.

**¹H NMR (500 MHz, CD₃CN):** δ = 10.05 (br s, 1H), 7.83 (d, *J* = 8.4 Hz, 1H), 7.48-7.54 (m, 2H), 7.46 (s, 1H), 7.36-7.45 (m, 3H), 7.27 (t, *J* = 8.3 Hz, 1H), 6.98 (d, *J* = 8.1 Hz, 1H), 5.91 (s, 2H), 5.41 (s, 2H), 3.26-3.35 (m, 2H), 3.13-3.20 (m, 2H), 2.88 (s, 3H), 2.87 (s, 3H), 1.14 (s, 9H) ppm. **¹³C NMR (126 MHz, CD₃CN):** δ = 178.4 (C_{q}, 1C), 161.0 (C_{q}, q, *J_{C-F}* = 35.8 Hz, 1C), 151.3 (C_{q}, 1C), 151.1 (C_{q}, 1C), 138.4 (C_{q}, 1C), 136.4 (C_{q}, 1C), 129.6 (CH, 2C), 129.6 (CH, 1C), 129.3 (CH, 2C), 126.8 (CH, 1C), 124.6 (CH, 1C), 120.3 (Cq, 1C), 117.4 (C_{q}, q, *J_{C-F}* = 291.2 Hz, 1C), 116.1 (Cq, 1C), 110.6 (CH, 1C), 107.3 (CH, 1C), 85.6 (CH₂, 1C), 69.6 (CH₂, 1C), 58.8 (CH₂, 1C), 43.5 (CH₃, 2C), 39.5 (C_{q}, 1C), 27.0 (CH₃, 3C), 22.7 (CH₂, 1C) ppm. **¹⁹F NMR (282 MHz, CD₃CN):** δ = -76.2 (s, 3F) ppm. **HR MS (ESI⁺):** m/z [M+H]⁺ calcd. for [C₂₆H₃₃N₂O₅]⁺: 453.2384, found 453.2388.

### 1-Benzyloxycarbonyl-4-n-butanoyloxymethyloxy-N,N-dimethyltryptammonium trifluoroacetate (13b)

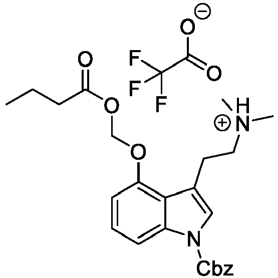

A solution of **10** (400 mg, 1.18 mmol, 1.00 eq) in anhydrous DMF (5.6 mL) was treated with NaH (60 % suspension in mineral oil, targeted, 70.9 mg, 1.77 mmol, 1.50 eq) at - 30 to -45 °C. After stirring for 30 min at this temperature, iodomethyl butyrate **12b** (404 mg, 1.77 mmol, 1.50 eq) as a solution in anhydrous THF was slowly added. The reaction mixture was allowed to slowly warm to room temperature and stirred for further 5 h. Subsequently, the reaction mixture was poured into aq. 1 N NaOH (5.4 mL) and extracted with ethyl acetate (3x20 mL). The organic layer was dried over Na₂SO₄, concentrated, dissolved in acetonitrile (+0.1 % TFA) and purified by means of reversed-phase chromatography on a preparative HPLC instrument (conditions I). Evaporation of volatiles followed by lyophilization afforded **13b** (96.8 mg, 171 µmol, 14 %) as a colorless oil.

**¹H NMR (500 MHz, CD₃CN):** δ = 10.74 (br s, 1H), 7.82 (d, *J* = 8.3 Hz, 1H), 7.50 (d, *J* = 7.1 Hz, 2H), 7.46 (s, 1H), 7.35-7.45 (m, 3H), 7.27 (t, *J* = 8.3 Hz, 1H), 6.95 (d, *J* = 8.1 Hz, 1H), 5.90 (s, 2H), 5.41 (s, 2H), 3.24-3.35 (m, 2H), 3.10-3.22 (m, 2H), 2.86 (s, 6H), 2.32 (t, *J* = 7.3 Hz, 2H), 1.57 (sxt, *J* = 7.4 Hz, 2H), 0.85 (t, *J* = 7.5 Hz, 3H) ppm. **¹³C NMR (126 MHz, CD₃CN):** δ = 173.7 (C_{q}, 1C), 161.2 (C_{q}, q, *J_{C-F}* = 35.1 Hz, 1C), 151.3 (C_{q}, 1C), 151.2 (C_{q}, 1C), 138.4 (C_{q}, 1C), 136.5 (C_{q}, 1C), 129.6 (CH, 2C), 129.6 (CH, 1C), 129.3 (CH, 2C), 126.8 (CH, 1C), 124.6 (CH, 1C), 121.1, 120.4 (C_{q}, 1C), 117.6 (C_{q}, q, *J_{C-F}* =292.5 Hz, 1C), 116.3 (C_{q}, 1C), 110.6 (CH, 1C), 107.3 (CH, 1C), 85.4 (CH₂, 1C), 69.6 (CH₂, 1C), 58.7 (CH₂, 1C), 43.3 (CH₃, 2C), 36.4 (CH₂, 1C), 22.7 (CH₂, 1C), 18.8 (CH₂, 1C), 13.7 (CH₃, 1C) ppm. **¹⁹F NMR (282 MHz, CD₃CN):** δ = -76.1 ppm. **HR MS (ESI⁺):** m/z [M+H]⁺ calcd. for [C25H31N2O5]⁺: 439.2227, found 439.2224.

### 1-Benzyloxycarbonyl-4-pivaloyloxymethyloxy-N-ethyl-N-methyltryptammonium trifluoroacetate (13c)

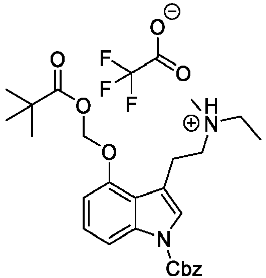

Under a nitrogen atmosphere, **11** (100 mg, 284 µmol, 1.00 eq) was dissolved in dry DMF (1.3 mL). After cooling to -40 to -50 °C, NaH (60 % dispersion in mineral oil, 17.0 mg, 426 µmol, 1.50 eq) was added slowly followed by a solution of iodomethyl pivalate **12a** (103 mg, 426 µmol, 1.50 eq) in dry THF (1.3 mL). The reaction mixture was allowed to warm to room temperature and stirring was continued for 2 h. Subsequently, the mixture was poured into 1 N NaOH (1.35 mL) and extracted with ethyl acetate (3x5 mL). The organic layer was dried over Na₂SO₄, concentrated, dissolved in acetonitrile (+0.1 % TFA) and purified by means of reversed-phase chromatography on a preparative HPLC instrument (conditions I). Evaporation of volatiles followed by lyophilization afforded **13c** (73.3 mg, 126 µmol, 44 %) as a colorless oil. **¹H NMR (500 MHz, CD₃CN):** δ = 9.57 (br s, 1H), 7.85 (d, *J* = 8.3 Hz, 1H), 7.49-7.55 (m, 3H), 7.34-7.47 (m, 3H), 7.30 (t, *J* = 8.3 Hz, 1H), 7.01 (d, *J* = 7.9 Hz, 1H), 5.93 (d, *J* = 6.7 Hz, 1H), 5.90 (d, *J* = 6.7 Hz, 1H), 5.43 (s, 2H), 3.34-3.40 (m, 1H), 3.09-3.30 (m, 5H), 2.83-2.86 (m, 3H), 1.28 (t, *J* = 7.3 Hz, 3H), 1.14 (s, 9H) ppm. **¹³C NMR (126 MHz, CD₃CN):** δ = 178.4 (C_{q}, 1C), 151.4 (C_{q}, 1C), 151.3 (C_{q}, 1C), 138.5 (C_{q}, 1C), 136.5 (C_{q}, 1C), 129.7 (CH, 2C), 129.6 (CH, 1C), 129.4 (CH, 2C), 126.9 (CH, 1C), 124.8 (CH, 1C), 120.4 (C_{q}, 1C), 116.2 (C_{q}, 1C), 110.6 (CH, 1C), 107.5 (CH, 1C), 85.8 (CH₂, 1C), 69.7 (CH₂, 1C), 56.5 (CH₂, 1C), 52.1 (CH₂, 1C), 39.9 (CH₃, 1C), 39.5 (C_{q}, 1C), 27.0 (CH₃, 3C), 22.5 (CH₂, 1C), 9.3 (CH₃, 1C) ppm. Trifluoroacetate counterion not detected due to low signal intensity caused by C-F coupling. **¹⁹F NMR (282 MHz, CD₃CN):** δ = -76.3 (s, 3F) ppm. **HR MS (ESI⁺):** m/z [M+H]⁺ calcd. for [C₂₇H₃₅N₂O₅]⁺: 467.2540, found 467.2549.

### 1-Benzyloxycarbonyl-4-n-butanoyloxymethyloxy-N-ethyl-N-methyltryptammonium trifluoroacetate (13d)

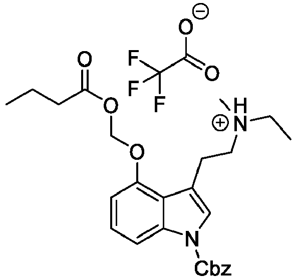

A solution of **11** (200 mg, 567 µmol, 1.00 eq) in anhydrous DMF (2.7 mL) was treated with NaH (60 % suspension in mineral oil, 34.1 mg, 851 µmol, 1.50 eq) at 0 °C. After 30 min at 0 °C, gas generation ceased and a clear brown solution was obtained. The temperature was then lowered to -25 °C to -30 °C, and **12b** (129 mg, 567 µmol, 1.00 eq) as a solution in anhydrous THF (2.7 mL) was slowly added. After completion of the addition, the reaction mixture was allowed to slowly warm to room temperature. After stirring for 4 h, the mixture was poured into aq. 1 N NaOH (2.7 mL) and extracted with ethyl acetate (3x10 mL). The organic layer was dried over Na₂SO₄, concentrated, dissolved in acetonitrile (+0.1 % TFA) and purified by means of reversed-phase chromatography on an preparative HPLC instrument (conditions I). Evaporation of volatiles followed by lyophilization afforded **13d** (133 mg, 234 µmol, 41 %) as an orange oil.

**¹H NMR (300 MHz, CD₃CN):** δ = 10.47 (br s, 1H), 7.85 (d, *J* = 8.2 Hz, 1H), 7.35-7.56 (m, 6H), 7.28 (t, *J* = 8.2 Hz, 1H), 6.97 (d, *J* = 8.1 Hz, 1H), 5.87-5.97 (m, 2H), 5.43 (s, 2H), 3.05-3.41 (m, 6H), 2.84 (d, *J* = 4.7 Hz, 3H), 2.33 (t, *J* = 7.3 Hz, 2H), 1.58 (sxt, *J* = 7.4 Hz, 2H), 1.29 (t, *J* = 7.3 Hz, 3H), 0.86 (t, *J* = 7.4 Hz, 3H) ppm. **¹³C NMR (75 MHz, CD₃CN):** δ = 173.6 (C_{q}, 1C), 151.4 (C_{q}, 1C), 151.3 (C_{q}, 1C), 138.4 (C_{q}, 1C), 136.5 (C_{q}, 1C), 129.6 (CH, 2C), 129.6 (CH, 1C), 129.3 (CH, 2C), 126.8 (CH, 1C), 124.7 (CH, 1C), 120.4 (C_{q}, 1C), 116.4 (C_{q}, 1C), 110.6 (CH, 1C), 107.4 (CH, 1C), 85.6 (CH₂, 1C), 69.6 (CH₂, 1C), 56.4 (CH₂, 1C), 51.8 (CH₂, 1C), 39.7 (CH₃, 1C), 36.4 (CH₂, 1C), 22.4 (CH₂, 1C), 18.8 (CH₂, 1C), 13.6 (CH₃, 1C), 9.3 (CH₃, 1C) ppm. Trifluoroacetate counterion not detected due to low signal intensity caused by C-F coupling. **¹⁹F NMR (282 MHz, CD₃CN):** δ = -76.1 (s, 3F) ppm. **HR MS (ESI⁺):** m/z [M+H]⁺ calcd. for [C₂₆H₃₃N₂O₅]⁺: 453.2384, found 453.2387.

### 4-Pivaloyloxymethyloxy-N,N-dimethyltryptammonium fumarate (15a)

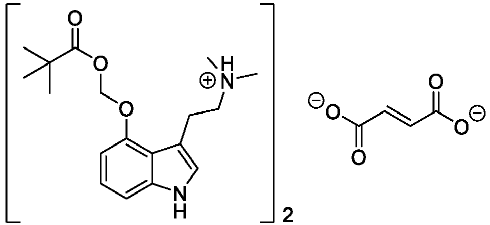

A 100 mL two-necked flask was charged with 10 % Pd/C (72.3 mg, 67.9 µmol, 0.12 eq). After three evacuation-backfill cycles with nitrogen, the flask was filled with hydrogen gas and equipped with a hydrogen-containing balloon. Then, a solution of **13a** (335 mg, 591 µmol, 1.00 eq) in EtOAc (37.5 mL) was added via syringe, and the reaction mixture was stirred at room temperature for 16 h. Subsequently, the reaction mixture was filtered through a pad of cotton. The filter cake was rinsed with EtOAc (3x5 mL) and concentrated by rotary evaporation. The crude was then dissolved in acetonitrile (ACN)/H₂O (1:1, +0.1 % TFA) and purified by means of reversed-phase chromatography on a preparative HPLC instrument (conditions II). The product fractions were concentrated by rotary evaporation, set to pH 10-11 using 1 N NaOH (8.7 mL) and extracted with dichloromethane (3x150 mL). Drying over Na₂SO₄ followed by rotary evaporation afforded a colorless oil. In order to remove residual traces of TFA which had been detected by ¹⁹F NMR spectroscopy, the oil was dissolved in 200 mL Et₂O, and the organic phase was washed with carbonate buffer (0.1 M, pH 10.6, 3x50 mL). Drying over Na₂SO₄ followed by evaporation of volatiles afforded 4-pivaloyloxymethyloxy-*N*,*N*-dimethyltryptamine **14a** (110 mg, 346 µmol, 59 %) as a colorless solid.

**¹H NMR (300 MHz, CD₃CN):** δ = 9.10 (br s, 1H), 7.05-7.09 (m, 1H), 6.98-7.05 (m, 1H), 6.94-6.98 (m, 1H), 6.68 (dd, *J* = 7.3 Hz, *J*' = 1.2 Hz, 1H), 5.87 (s, 2H), 2.86-3.00 (m, 2H), 2.46-2.55 (m, 2H), 2.23 (s, 6H), 1.16 (s, 9H) ppm. **¹³C NMR (75 MHz, (CD₃)₂CO):** δ = 177.4 (C_{q}, 1C), 152.3 (C_{q}, 1C), 139.7 (C_{q}, 1C), 122.7 (CH, 1C), 122.5 (CH, 1C), 119.0 (C_{q}, 1C), 114.4 (C_{q}, 1C), 107.2 (CH, 1C), 102.9 (CH, 1C), 86.2 (CH₂, 1C), 62.4 (CH₂, 1C), 45.7 (CH₃, 2C), 39.4 (C_{q}, 1C), 27.2 (CH₃, 3C), 25.7 (CH₂, 1C) ppm. **HR MS (ESI⁺):** m/z [M+Na]⁺ calcd. for [C₁₈H₂₆N₂NaO₃]⁺: 341.1836, found 341.1835; [M+H]⁺ calcd. for [C₁₈H₂₇N₂O₃]⁺: 319.2016, found 319.2020. The analytical data correspond to the ones previously reported in the literature.^{[11]}

To a solution of fumaric acid (19.4 mg, 166 µmol, 0.48 eq) in acetone (3.0 mL), **14a** (110 mg, 346 µmol, 1.00 eq) dissolved in acetone (1.9 mL) was added and the obtained mixture was thoroughly vortexed. After standing for 1.5 h at 5°C, the suspension was centrifuged (3,500 RCF, 5 min) and decanted. The residue was washed with cold (5 °C) acetone (2 × 0.8 mL) and lyophilized, which afforded **15a** (106 mg, 282 µmol, 82 %) as a colorless solid.

**¹H NMR (500 MHz, (CD₃)₂SO):** δ = 10.89 (br s, 1H), 7.05 (d, *J* = 2.1 Hz, 1H), 7.01-7.03 (m, 1H), 6.96-7.00 (m, 1H), 6.63 (d, *J* = 7.5 Hz, 1H), 6.48 (s, 1H), 5.89 (s, 2H), 2.91-2.98 (m, 2H), 2.67-2.74 (m, 2H), 2.39 (s, 6H), 1.12 ppm (s, 9H) ppm. **¹³C NMR (75 MHz, (CD₃)₂SO):** δ = 176.7 (C_{q}, 1C), 167.4 (C_{q}, 1C), 150.5 (C_{q}, 1C), 138.3 (C_{q}, 1C), 134.9 (CH, 1C), 122.5 (CH, 1C), 121.6 (CH, 1C), 117.3 (C_{q}, 1C), 111.2 (C_{q}, 1C), 106.5 (CH, 1C), 101.7 (CH, 1C), 85.2 (CH₂, 1C), 60.1 (CH₂, 1C), 44.0 (CH₃, 2C), 38.4 (C_{q}, 1C), 26.6 (CH₃, 3C), 23.4 (CH₂, 1C) ppm. **HR MS (ESI⁺):** m/z [M+Na]⁺ calcd. for [C₁₈H₂₆N₂NaO₃]⁺: 341.1836, found 341.1829; [M+H]⁺ calcd. for [C₁₈H₂₇N₂O₃]⁺: 319.2016, found 319.2014.

### 4-n-Butanoyloxymethyloxy-N,N-dimethyltryptammonium fumarate (15b)

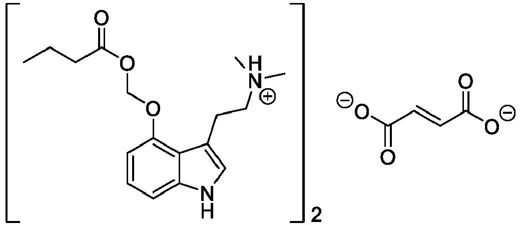

A 50 mL two-necked flask was charged with 10 % Pd/C (26.2 mg, 24.7 µmol, 0.15 eq). After three evacuation-backfill cycles with nitrogen, the flask was filled with hydrogen gas and equipped with a hydrogen-containing balloon. Then, a solution of **13b** (90.1 mg, 163 µmol, 1.00 eq) in EtOAc (13 mL) was added via syringe, and the reaction mixture was stirred at room temperature for 6 h. Subsequently, the reaction mixture was filtered through a pad of cotton. The filter cake was rinsed with EtOAc (3x2 mL) and concentrated by rotary evaporation. The crude was then dissolved in ACN/H₂O (1:1, +0.1 % TFA) and purified by means of reversed-phase chromatography on a preparative HPLC instrument (conditions II). The product fractions were concentrated by rotary evaporation, set to pH 10-11 using 1 N NaOH (2.9 mL) and extracted with dichloromethane (3x45 mL). Drying over Na₂SO₄ followed by rotary evaporation afforded 4-*n*-butanoyloxymethyloxy-*N*,*N*-dimethyltryptamine **14b** (40.4 mg, 133 µmol, 81 %) as an impure yellowish oil which was used in the next step without further purification.

**¹H NMR (500 MHz, CD₃CN):** δ = 9.32 (br s, 1H), 7.04-7.09 (m, 1H), 6.98-7.04 (m, 1H), 6.96 (d, *J* = 1.7 Hz, 1H), 6.66 (d, *J* = 7.6 Hz, 1H), 5.86 (s, 2H), 2.92-3.01 (m, 2H), 2.50-2.59 (m, 2H), 2.32 (t, *J* = 7.3 Hz, 2H), 2.21-2.29 (m, 6H), 1.60 (sxt, *J* = 7.4 Hz, 2H), 0.89 (t, *J* = 7.4 Hz, 3H) ppm.

To a solution of fumaric acid (6.65 mg, 56.7 µmol, 0.43 eq) in acetone (1.4 mL), **14b** (40.4 mg, 133 µmol, 1.00 eq) dissolved in acetone (0.8 mL) was added and the obtained mixture was thoroughly vortexed. After standing for 6 h at 5 °C, the suspension was centrifuged (3,500 RCF, 5 min) and decanted. The residue was washed with cold (5°C) acetone (2 × 0.3 mL) and lyophilized, which afforded **15b** (33.2 mg, 91.6 µmol, 69 %) as a colorless solid.

**¹H NMR (500 MHz, (CD₃)₂SO):** δ = 10.92 (br s, 1H), 7.07 (d, *J* = 1.7 Hz, 1H), 7.01-7.05 (m, 1H), 6.95-7.01 (m, 1H), 6.63 (d, *J* = 7.6 Hz, 1H), 6.50 (s, 1H), 5.89 (s, 2H), 2.92-3.03 (m, 2H), 2.73-2.86 (m, 2H), 2.46 (s, 6H), 2.34 (t, *J* = 7.3 Hz, 2H), 1.54 (sxt, *J* = 7.3 Hz, 2H), 0.84 (t, *J* = 7.4 Hz, 3H) ppm. **¹³C NMR (126 MHz, (CD₃)₂SO):** δ = 172.1 (C_{q}, 1C), 167.5 (C_{q}, 1C), 150.4 (C_{q}, 1C), 138.3 (C_{q}, 1C), 134.9 (CH, 1C), 122.6 (CH, 1C), 121.6 (CH, 1C), 117.2 (C_{q}, 1C), 110.8 (C_{q}, 1C), 106.4 (CH, 1C), 101.6 (CH, 1C), 84.8 (CH₂, 1C), 59.7 (CH₂, 1C), 43.6 (CH₃, 2C), 35.3 (CH₂, 1C), 23.0 (CH₂, 1C), 17.7 (CH₂, 1C), 13.2 (CH₃, 1C) ppm. **HR MS (ESI⁺):** m/z [M+Na]⁺ calcd. for [C₁₇H₂₄N₂NaO₃]⁺: 327.1679, found 327.1667; [M+H]⁺ calcd. for [C₁₇H₂₅N₂O₃]⁺. 305.1860, found 305.1852.

### 4-Pivaloyloxymethyloxy-N-ethyl-N-methyltryptammonium fumarate (15c)

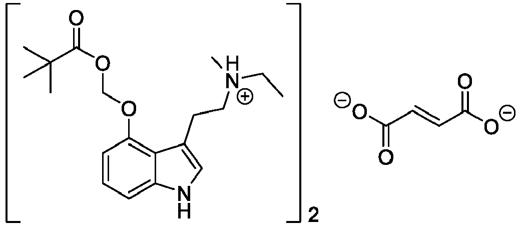

A25 mL two-necked flask was charged with 10 % Pd/C (15.0 mg, 14.1 µmol, 0.12 eq). After three evacuation-backfill cycles with nitrogen, the flask was filled with hydrogen gas and equipped with a hydrogen-containing balloon. Then, a solution of **13c** (71.1 mg, 122 µmol, 1.00 eq) in EtOAc (7.8 mL) was added via syringe, and the reaction mixture was stirred at room temperature for 4.5 h. Subsequently, the reaction mixture was filtered through a pad of cotton. The filter cake was rinsed with EtOAc (3x1 mL) and concentrated by rotary evaporation. The crude was then dissolved in ACN/H₂O (1:1, +0.1 % TFA) and purified by means of reversed-phase chromatography on a preparative HPLC instrument (conditions II). The product fractions were concentrated by rotary evaporation, set to pH 10-11 using 1 N NaOH (1.8 mL) and extracted with dichloromethane (3x30 mL). Drying over Na₂SO₄ followed by rotary evaporation afforded a colorless oil. In order to remove residual traces of TFA which had been detected by ¹⁹F NMR spectroscopy, the oil was dissolved in 20 mL Et₂O, and the organic phase was washed with carbonate buffer (0.1 M, pH 10.6, 3x10 mL). Drying over Na₂SO₄ followed by evaporation of volatiles afforded 4-pivaloyloxymethyloxy-N-ethyl-*N*-methyltryptamine **14c** (28.0 mg, 84.2 µmol, 69 %) as a colorless oil.

**¹H NMR (300 MHz, (CD₃)₂CO):** δ = 10.08 (br s, 1H), 6.95-7.10 (m, 3H), 6.66-6.73 (m, 1H), 5.94 (s, 2H), 2.97-3.04 (m, 2H), 2.59-2.70 (m, 2H), 2.48 (q, *J* = 7.1 Hz, 2H), 2.30 (s, 3H), 1.18 (s, 9H), 1.05 (t, *J* = 7.2 Hz, 3H) ppm.

To a solution of fumaric acid (4.89 mg, 42.1 µmol, 0.50 eq) in acetone (0.6 mL), **14c** (28.0 mg, 84.2 µmol, 1.00 eq) dissolved in acetone (0.9 mL) was added and the obtained mixture was thoroughly vortexed. After standing for 1.5 h at 5°C, the suspension was centrifuged (3,500 RCF, 5 min) and decanted. The residue was washed with cold (5°C) acetone (2 × 0.2 mL) and lyophilized, which afforded **15c** (25.8 mg, 66.1 µmol, 78 %) as a colorless crystalline solid.**¹H NMR (500 MHz, (CD₃)₂SO):** δ = 10.90 (br s, 1H), 7.06 (d, *J* = 1.8 Hz, 1H), 7.01-7.04 (m, 1H), 6.96-7.01 (m, 1H), 6.63 (d, *J* = 7.3 Hz, 1H), 6.47 (br s, 1H), 5.89 (s, 2H), 2.91-3.00 (m, 2H), 2.73-2.82 (m, 2H), 2.61-2.72 (m, 2H), 2.42 (s, 3H), 1.12 (s, 9H), 1.07 (t, *J* = 7.2 Hz, 3H) ppm. **¹³C NMR (126 MHz, (CD₃)₂SO):** δ = 176.6 (C_{q}, 1C), 167.4 (C_{q}, 1C), 150.5 (C_{q}, 1C), 138.3 (C_{q}, 1C), 134.9 (CH, 1C), 122.7 (CH, 1C), 121.6 (CH, 1C), 117.2 (C_{q}, 1C), 110.8 (C_{q}, 1C), 106.5 (CH, 1C), 101.7 (CH, 1C), 85.2 (CH₂, 1C), 57.3 (CH₂, 1C), 50.2 (CH₂, 1C), 40.1 (CH₃, 1C), 38.4 (C_{q}, 1C), 26.6 (CH₃, 3C), 22.7 (CH₂, 1C), 10.7 (CH₃, 1C) ppm. **HR MS (ESI⁺):** m/z [M+H]⁺calcd. for [C₁₉H₂₉N₂O₃]⁺: 333.2173, found 333.2175.

### 4-n-Butanoyloxymethyloxy-N-ethyl-N-methyltryptammonium fumarate (15d)

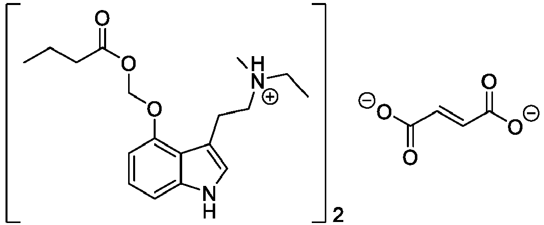

A 50 mL two-necked flask was charged with 10 % Pd/C (28.0 mg, 26.3 µmol, 0.12 eq). After three evacuation-backfill cycles with nitrogen, the flask was filled with hydrogen gas and equipped with a hydrogen-containing balloon. Then, a solution of **13d** (133 mg, 234 µmol, 1.00 eq) in EtOAc (14.5 mL) was added via syringe, and the reaction mixture was stirred at room temperature for 4.5 h. Subsequently, the reaction mixture was filtered through a pad of cotton. The filter cake was rinsed with EtOAc (3x2 mL) and concentrated by rotary evaporation. The crude was then dissolved in ACN/H₂O (1:1, +0.1 % TFA) and purified by means of reversed-phase chromatography on a preparative HPLC instrument (conditions II). The product fractions were concentrated by rotary evaporation, set to pH 10-11 using 1 N NaOH (3.3 mL) and extracted with dichloromethane (3x50 mL). Drying over Na₂SO₄ followed by rotary evaporation afforded a colorless oil. In order to remove residual traces of TFA which had been detected by ¹⁹F NMR spectroscopy, the oil was dissolved in 40 mL Et₂O, and the organic phase was washed with carbonate buffer (0.1 M, pH 10.6, 3x20 mL). Drying over Na₂SO₄ followed by evaporation of volatiles afforded 4-*n*-butanoyloxymethyloxy-*N*-ethyl-*N*-methyltryptamine **14d** (49.6 mg, 156 µmol, 68 %) as a colorless oil.

**¹H NMR (300 MHz, (CD₃)₂CO):** δ = 10.44 (br s, 1H), 6.93-7.11 (m, 3H), 6.66 (d, *J* = 7.6 Hz, 1H), 5.93 (s, 2H), 2.92-3.05 (m, 2H), 2.58-2.70 (m, 2H), 2.40-2.54 (m, 2H), 2.35 (t, *J* = 7.3 Hz, 2H), 2.26-2.31 (m, 3H), 1.62 (sxt, *J* = 7.4 Hz, 2H), 0.99-1.10 (m, 3H), 0.91 (t, *J* = 7.4 Hz, 3H) ppm.

To a solution of fumaric acid (9.04 mg, 77.9 µmol, 0.50 eq) in acetone (1.7 mL), **14d** (49.6 mg, 156 µmol, 1.00 eq) dissolved in acetone (0.9 mL) was added and the obtained mixture was thoroughly vortexed. After standing for 1.5 h at 5 °C, the suspension was centrifuged (3,500 RCF, 5 min) and decanted. The residue was washed with cold (5 °C) acetone (2 × 0.4 mL) and lyophilized, which afforded **15d** (39.2 mg, 104 µmol, 67 %) as a colorless solid.

**¹H NMR (500 MHz, (CD₃)₂SO):** δ = 10.93 (s, 1H), 7.07 (d, *J* = 2.2 Hz, 1H), 7.03 (dd, *J* = 8.1 Hz, *J* = 0.7 Hz, 1H), 6.98 (t, *J* = 7.8 Hz, 1H), 6.63 (dd, *J* = 7.6 Hz, J=0.6 Hz, 1H), 6.47 (s, 1H), 5.89 (s, 2H), 2.91-3.02 (m, 2H), 2.77-2.85 (m, 2H), 2.71 (q, *J* = 7.2 Hz, 2H), 2.45 (s, 3H), 2.34 (t, *J* = 7.2 Hz, 2H), 1.54 (sxt, *J* = 7.3 Hz, 2H), 1.09 (t, *J* = 7.2 Hz, 3H), 0.84 ppm (t, *J* = 7.4 Hz, 3H) ppm. **¹³C NMR (126 MHz, (CD₃)₂SO):** δ = 172.1 (C_{q}, 1C), 167.8 (C_{q}, 1C), 150.5 (C_{q}, 1C), 138.3 (C_{q}, 1C), 135.1 (CH, 1C), 122.6 (CH, 1C), 121.6 (CH, 1C), 117.2 (C_{q}, 1C), 111.1 (C_{q}, 1C), 106.5 (CH, 1C), 101.6 (CH, 1C), 84.8 (CH₂, 1C), 57.5 (CH₂, 1C), 50.1 (CH₂, 1C), 40.0 (CH₃, 1C), 35.3 (CH₂, 1C), 22.7 (CH₂, 1C), 17.7 (CH₂, 1C), 13.3 (CH₃, 1C), 10.8 (CH₃, 1C) ppm. **HR MS (ESI⁺):** m/z [M+H]⁺ calcd. for [C₁₈H₂₇N₂O₃]⁺: 319.2016, found 319.2026.

### 4-Pivaloyloxymethyloxy-N-ethyl-N-methyltryptammonium mesylate (16)

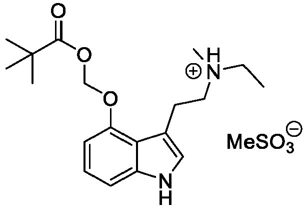

A25 mL two-necked flask was charged with 10 % Pd/C (15.0 mg, 14.1 µmol, 0.12 eq). After three evacuation-backfill cycles with nitrogen, the flask was filled with hydrogen gas and equipped with a hydrogen-containing balloon. Then, a solution of **13c** (71.1 mg, 122 µmol, 1.00 eq) in EtOAc (7.8 mL) was added via syringe, and the reaction mixture was stirred at room temperature for 4.5 h. Subsequently, the reaction mixture was filtered through a pad of cotton. The filter cake was rinsed with EtOAc (3x1 mL) and concentrated by rotary evaporation. The crude was then dissolved in ACN/H₂O (1:1, +0.1 % TFA) and purified by means of reversed-phase chromatography on a preparative HPLC instrument (conditions II). The product fractions were concentrated by rotary evaporation, set to pH 10-11 using 1 N NaOH (1.8 mL) and extracted with dichloromethane (3x30 mL). Drying over Na₂SO₄ followed by rotary evaporation afforded a colorless oil. In order to remove residual traces of TFA which had been detected by ¹⁹F NMR spectroscopy, the oil was dissolved in 20 mL Et₂O, and the organic phase was washed with carbonate buffer (0.1 M, pH 10.6, 3x10 mL). Drying over Na₂SO₄ followed by evaporation of volatiles afforded 4-pivaloyloxymethyloxy-*N*-ethyl-*N*-methyltryptamine **14c** (28.0 mg, 84.2 µmol, 69 %) as a colorless oil.

**¹H NMR (300 MHz, (CD₃)₂CO):** δ = 10.08 (br s, 1H), 6.95-7.10 (m, 3H), 6.66-6.73 (m, 1H), 5.94 (s, 2H), 2.97-3.04 (m, 2H), 2.59-2.70 (m, 2H), 2.48 (q, *J* = 7.1 Hz, 2H), 2.30 (s, 3H), 1.18 (s, 9H), 1.05 (t, *J* = 7.2 Hz, 3H) ppm.

**14c** (19.7 mg, 59.3 µmol, 1.00 eq) was then dissolved in acetone (0.8 mL) and added to a solution of methanesulfonic acid (3.89 µl, 5.69 mg, 59.3 µmol, 1.00 eq) in acetone (0.2 mL), followed by cyclohexane (3.0 mL). The mixture was thoroughly vortexed, allowed to stand at 5 °C for 2 h, then centrifuged (3,500 RCF, 5 min) and decanted. The residue was washed with cold (5 °C) cyclohexane (2 × 0.5 mL) and lyophilized, which afforded **16** (16.4 mg, 38.3 µmol, 65 %) as a yellowish oil.

**¹H NMR (500 MHz, (CD₃)₂SO):** δ = 11.04 (br s, 1H), 9.03-9.24 (m, 2H), 7.18 (d, *J* = 2.3 Hz, 1H), 6.98-7.11 (m, 2H), 6.70 (dd, *J* = 7.3 Hz, *J* = 1.1 Hz, 1H), 5.87-5.99 (m, 2H), 3.02-3.30 (m, 6H), 2.85 (d, *J* = 5.0 Hz, 2H), 2.30 (s, 2H), 1.24 (t, *J* = 7.2 Hz, 3H), 1.12 (s, 7H) ppm. **¹³C NMR (126 MHz, (CD₃)₂SO):** δ = 176.9 (C_{q}, 1C), 150.1 (C_{q}, 1C), 138.4 (C_{q}, 1C), 123.5 (CH, 1C), 122.0 (CH, 1C), 116.8 (C_{q}, 1C), 108.2 (C_{q}, 1C), 106.6 (CH, 1C), 101.7 (CH, 1C), 85.1 (CH₂, 1C), 55.7 (CH₂, 1C), 50.3 (CH₂, 1C), 38.8 (CH₃, 1C), 38.4 (C_{q}, 1C), 26.6 (CH₃, 3C), 21.4 (CH₂, 1C), 8.9 (CH₃, 1C) ppm. **HR MS (ESI⁺):** m/z [M+H]⁺ calcd. for [C₁₉H₂₉N₂O₃]⁺: 333.2173, found 333.2179.

### Characterization of compound properties

### Passive membrane permeability and cell viability

**Table 5: Passive membrane permeability Pₑ, mass retention R, and viability of human hepatocyte-derived cells (HuH-7) after incubation with the respective prodrug. All values are the mean of triplicates ± standard deviation unless otherwise stated.**

| **Compound** | | **Pₑ [10⁻⁶ cm/s]** | **R [%]** | **Cell Viability in HuH7 at 50 µM [%]** | |
|---|---|---|---|---|---|
| | | | | **2 h Incubation** | **24 h Incubation** |
| **Carboxylic ester prodrugs** | **7a** | n.d. | n.d. | 96.9 ± 1.6 | 81.9 ± 2.8 |
| | **7b** | n.d. | n.d. | 104.3 ± 1.9 | 80.0 ± 4.8 |
| | **7c** | n.d. | n.d. | 104.4 ± 4.5 | 83.8 ± 1.7 |
| | **8a^{a}** | 5.6 ± 0.8 | 4 | 88.7 ± 3.0 | 85.4 ± 3.6 |
| | **8b** | 9.2 ± 0.8 | 4 | 88.1 ± 6.0 | 83.7 ± 1.0 |
| | **8c^{a}** | 11.8 ± 1.0 | 7 | 30.2 ± 1.0 | 74.3 ± 8.0 |
| | **8d** | 9.5 ± 0.7 | 4 | 91.4 ± 3.7 | 82.1 ± 2.3 |
| | **8e** | n.d. | n.d. | 101.2 ± 3.1 | 81.7 ± 2.0 |
| | **8f** | 10.7 ± 0.7 | -2 | 80.4 ± 2.1 | 79.4 ± 2.1 |
| | **8g** | 10.6 ± 0.8 | 2 | 81.7 ± 8.6 | 79.8 ± 4.6 |
| | **8h** | 11.2 ± 0.8 | 1 | 28.7 ± 1.1 | 42.3 ± 25.9 |
| | **8i** | 9.4 ± 0.8 | 6 | 95.0 ± 3.8 | 80.1 ± 1.7 |
| | **8j** | n.d. | n.d. | 89.6 ± 3.4 | 78.0 ± 2.0 |
| **Acyloxymethyl prodrugs** | **15a** | 9.1 ± 0.5 | 4 | 61.6 ± 6.0 | 61.6 ±18.4 |
| | **15b** | 8.1 ± 0.8^{b} | 7^{b} | 90.0 ± 5.2 | 79.3 ± 3.5 |
| | **15c** | 9.7 ± 0.6 | 2 | 48.3 ± 16.1 | 47.1 ± 10.4 |
| | **15d** | 8.0 ± 0.3 | 6 | 85.4 ± 2.7 | 80.6 ± 3.3 |
| ***N*-Cbz derivatives** | **10** | n.d. | n.d. | 0.1 ± 0.2 | -1.3 ± 0.1 |
| | **11** | n.d. | n.d. | 0.1 ± 0.2 | -2.1 ± 0.1 |
| **References** | **Acetylsalicylic acid** | n.d. | n.d. | 92.9 ± 3.3 | 83.8 ± 3.8 |
| | **Caffeine** | 12.8 ± 0.9^{c} | 1 | 109.8 ± 3.3 | 85.4 ± 0.9 |
| | **Furosemide** | -0.4 ± 0.3^{c} | 2 | 121.9 ± 13.8 | 90.7 ± 1.1 |
| | **Imipramine HCl** | 12.4 ± 1.0^{c} | 21 | 91.1 ± 3.7 | 24.4 ± 5.3 |
| | **Phenytoin** | 7.5 ± 1.0^{c} | 57 | 122.9 ± 1.7 | 84.2 ± 2.7 |
| | **Psilocin 1** | n.d. | n.d. | 93.7 ± 6.3 | 72.5 ± 8.3 |
| | **4-OH-MET fumarate 17** | 11.0 ± 1.0 | 29 | 91.0 ± 1.7 | 82.9 ± 1.3 |

| | | | | | |
|---|---|---|---|---|---|
| ^{a} Reference examples. ^{b} Mean of six replicates. ^{c} literature: cf. Chen et al. Pharm. Res. 2008, 15, 1511: Pₑ(Caffeine) = (9.89 ± 1.52)·10⁻⁶cm/s, Pₑ(Furosemide) = (0.46 ± 0.06)·10⁻⁶ cm/s, Pₑ(Imipramine HCl) = (10.11 ± 0.30)·10⁻⁶ cm/s, Pₑ(Phenytoin) = (5.73 ± 0.53)·10⁻⁶ cm/s. | | | | | |

Table 5 shows the passive membrane permeability and mass retention of the synthesized compounds in comparison to *N*-Cbz precursors and reference compounds. As can be seen, a high membrane permeability (> 1.5·10⁻⁶ cm/s) was found for all investigated prodrugs. Regarding Pₑ, a value of at least 5 × 10⁻⁶ cm/s can be considered to be preferable and is fulfilled by all measured prodrugs. The more preferable values of at least 8 × 10⁻⁶ cm/s are achieved by compounds **8b** and **8d**, **8f** to **8i**, and **15a** to **15d**. Further, the highest Pₑ measured can be observed for compounds **8f** to **8h**, in which R₃ of formula (1) is one of *tert*-butyl, isobutyl, and neopentyl, due to their lipophilicity.

Regarding cell viability of HuH-7 cells, generally, incubation with prodrugs resulted in a similar cell viability as incubation with the parent drugs **1** and **17**. In addition, data obtained after incubating for 2 h show the same trends as data obtained after 24 h incubation except in the case of reference compound **8c**. As already mentioned above, high lipophilicity and high passive membrane permeability of the prodrugs inversely correlate with cell viability. The *N*-carbamates **10** and **11** were found to be extremely toxic for HuH-7 cells, likely due to the combination of a relatively high lipophilicity and a phenolic pKₐ closer to 6.3. Aliphatic promoieties R₃ comprising acyl residues with three or four carbon atoms, along with aromatic promoieties R₃, represent a good compromise between passive membrane permeability and cellular tolerability. In view of cell viability, compounds **8b**, **7a/8d**, **7b**, **7c/8e** and **8i,** as well as **15d** are most preferred, in which R₃ of formula (1) is one of *n*-propyl, isopropyl, 2-furyl, cyclopropyl and phenyl.

### Stability in human saliva and human blood plasma

**Table 6: Prodrug cleavage in human saliva and human plasma. All values are the mean of triplicates ± standard deviation unless otherwise stated.**

| **Compound** | | **t_{1/2} [min] at 37 °C and [prodrug]₀ = 50 µM** | | |
|---|---|---|---|---|
| | | **Human Saliva^{a}** | **10 % Human Plasma** | **100 % Human Plasma** |
| **Carboxylic ester prodrugs** | **7a** | 15-60 | 0.36 ± 0.02^{e} | n.d. |
| | **7b** | 15-60 | n.d. | < 0.1^{e} |
| **Carboxylic ester prodrugs** | **7c** | > 60 | n.d. | n.d. |
| | **8a^{b}** | 15-60 | 0.69 ± 0.14 | n.d. |
| | **8b** | 15-60 | 0.64 ± 0.04 | n.d. |
| | **8c^{b}** | < 15 | 0.66 ± 0.13 | n.d. |
| | **8d** | 15-60 | 0.44 ± 0.03 | n.d. |
| | **8e** | > 60 | n.d. | 0.84 ± 0.11^{c} |
| | **8f** | > 60 | n.d. | > 60^{e} |
| | **8g** | n.d. | > 8^{e} | n.d. |
| | **8h** | n.d. | > 8^{e} | n.d. |
| | **8i** | 15-60 | 1.5 ± 0.5^{d} | n.d. |
| | **8j** | 15-60 | n.d. | 0.14 ± 0.01 |
| **Acyloxymethyl prodrugs** | **15a** | 15-60 | n.d. | > 300 min^{d,e} |
| | **15b** | < 15 | 3.5 ± 0.8^{d} | 0.48 ± 0.10 |
| | **15c** | < 15 | n.d. | > 300 min^{e} |
| | **15d** | < 15 | 4.2 ± 0.1^{d} | n.d. |
| ***N*-Cbz derivatives** | **10** | n.d. | n.d. | > 5700^{d,e} |
| | **11** | n.d. | n.d. | > 5700^{d,e} |
| **Reference** | **Acetylsalicylic acid** | > 60 | n.d. | 146 ± 19^{f} |

| | | | | |
|---|---|---|---|---|
| ^{a} Mean of three measurements performed with fresh samples of three individuals. ^{b} Reference examples. ^{c} Two measurements. ^{d} No clear distinction between zero and first order kinetics possible. ^{e} No replicates. ^{f} [ASS]₀ = 560 µM; cf. Harthon et al. Acta Pharmacologica et Toxicologica 1971, 29, 155: (130 ± 48) min. | | | | |

Referring to Table 6, the behavior of the prodrugs in human blood plasma has been studied. The rate of prodrug depletion in diluted (10 %) and undiluted (100 %) human blood plasma at 37 °C was investigated by HPLC-UV analysis of aliquots that were quenched after appropriate time intervals. Starting from an initial prodrug concentration of 50 µM, prodrug depletion was detectable within seconds to minutes except for prodrugs bearing sterically extremely hindered acyl residues (**8f-h**, **15a**, **15c**). Where applicable, half-lives were determined from semi-logarithmic plots of the raw data. These half-lives are considered to reflect the cleavage activity associated with plasma esterases or other plasma-specific factors, because non-catalyzed hydrolysis in the PBS buffer used for plasma dilution was found to proceed significantly slower, even at 80 °C (Table 7).

**Table 7: Stability of the prodrugs in PBS at 80 °C.**

| **Compound** | | **t_{1/2} [min] in PBS, 80 °C [prodrug]₀** = **50 µM** |
|---|---|---|
| **Carboxylic ester prodrugs** | **7a** | n.d. |
| | **7b** | n.d. |
| | **7c** | n.d. |
| | **8a^{a}** | 34.0 ± 0.7 |
| | **8b** | 82.0 ± 1.6 |
| | **8c^{a}** | 69.5 ± 1.2 |
| | **8d** | 137 ± 1 |
| | **8e** | n.d. |
| | **8f** | 738 ± 16 |
| | **8g** | 321 ± 4 |
| | **8h** | 1354 ± 66 |
| | **8i** | 58.4 ± 1.1 |
| | **8j** | n.d. |
| **Acyloxymethyl prodrugs** | **15a** | 458 ± 23 |
| | **15b** | 58.6 ± 3.5 |
| | **15c** | 534 ± 12 |
| | **15d** | 73.3 ± 1.2 |
| ***N*-Cbz derivatives** | **10** | n.d. |
| | **11** | n.d. |
| **References** | **Psilocin 1** | n.d. |
| | **4-HO-MET fumarate 17** | 116 ± 4 |
| | **Acetylsalicylic acid** | n.d. |

| | | |
|---|---|---|
| ^{a} Reference examples. | | |

Again, referring to Table 6, while the nature of the acyl residue turned out to have a major influence on the cleavage kinetics, the substitution pattern of the aminoethyl side chain appears less relevant (cf. **7a** vs. **8d**, **7b** vs. **8j**, **15b** vs. **15d**). Hence, the relative cleavage kinetics which are observed for the 4-OH-MET prodrugs can be expected to be equally valid for prodrugs that bear other substituents at the sidechain nitrogen. In particular, the 4-OH-MET prodrugs can therefore be considered valid surrogates for the very closely related psilocin. The isobutyrate esters of 4-OH-MET **8d** and of psilocin **7a** were identified as fastest-metabolized prodrug in human plasma within the studied library. The 2-furoyl and in particular the cyclopropanecarboxylic ester analogs of 4-OH-MET **8j** and **8e** and of psilocin (**7b** and **7c**, respectively) were shown to have slower drug release kinetics.

With respect to sublingual administration, the prodrug stability in human whole saliva freshly collected from three caucasian individuals with an initial prodrug concentration of 50 µM) has further been tested (Table 6). Due to the large inter-individual differences of salivary esterase activity (cf. e.g., Maria et al. RSC Adv. 2020, 10, 24352), the mean half-lives obtained were used as rough estimates for prodrug stability and classified into three groups (< 15 min/ 15-60 min / > 60 min). As expected, acyloxymethyl prodrugs were metabolized faster in saliva than carboxylic ester prodrugs (**8b** vs. **15d**, **8f** vs. **15c**) while, surprisingly, the inverse trend was found in human plasma. Only few prodrugs (**7c**, **8e**, **8f**, R₃ = cyclopropyl, cyclopropyl, and *tert*-butyl, respectively) were found to have very high stability in human whole saliva (t_{1/2} > 60 min). With respect to the short duration of the sublingual administration and the excellent permeability of the parent drugs psilocin and 4-HO-MET, prodrugs with average half-lives above 15 min can be regarded as sufficiently stable for sublingual administration.

### Stability of prodrugs in methanol

The stability of the exemplary carboxylic ester **8b** in methanol was studied by dissolving a few milligrams of the fumarate ester in CD₃OD at ambient temperature and measuring ¹H NMR spectra in appropriate time intervals. Using the singlet originating from the fumarate anion (6.68 ppm) as an internal reference, the obtained spectra are quantitatively comparable. This analysis revealed that approximately 50 % of the initial amount of ester degraded within 18 days (Scheme 3), exemplifying the lability of carboxylic hydroxytryptamine esters in the presence of nucleophiles.

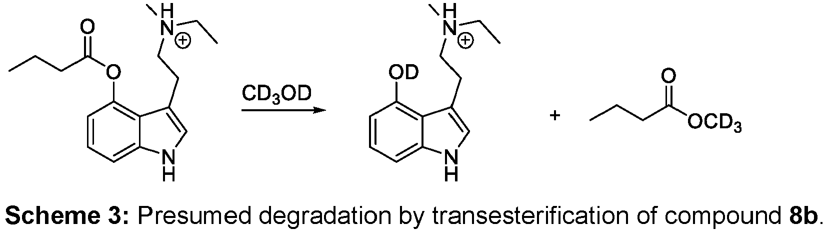

### Equilibrium solubility of prodrugs

For each reported solubility result, one shake-flask experiment was carried out unless otherwise stated. Measurements at pH 2.0 were performed in 0.01 M HCl. Measurements at pH 6.8 were performed in a KH₂PO₄/K₂HPO₄ buffer with an ionic strength of 70 mM. For each experiment, the solid, HPLC-purified sample (1.7 - 25 mg) was suspended in 250 - 500 µL of the aqueous buffer in a glass vial. The solution containing solid excess of the sample was then capped and shaken at 220 rpm in an Infors HT Minitron incubator at a defined temperature for 6 hours. Three aliquots (3 × 70 µL) were removed with a fine pipette from each shake-flask experiment, and the saturated solution in these aliquots was separated from precipitate by centrifugation (10000 rpm, 5 min) at the assay temperature. The clear supernatant (50 µL) was decanted, diluted with H₂O (+0.1 % TFA) as indicated below, and the concentration of sample in each aliquot was measured by HPLC-UV using calibrations curves (6-8 points, R²>0.99). For this, 40 µL of the diluted aliquots were injected into the HPLC system equipped with a ReproSil-Pur 120 ODS-3 column (5 µm, 50 × 2 mm, Dr. Maisch GmbH), eluted with mixtures of ACN and H2O (+0.1 % TFA) and a suitable gradient, and detected at 286 nm (**7a**, **7b**, **7c**, **19c**) or 244 nm (furosemide). The reported values are the mean of three measured concentrations. The error was estimated based on the standard deviation of the three measurements and the uncertainty of the calibration curve. Results are provided in Table 8.

**Table 8: Equilibrium solubility of selected prodrugs along with references.**

| **Compound** | **pH** | **Temperature [°C]** | **Dilution Factor** | **Equilibrium Solubility** | |
|---|---|---|---|---|---|
| | | | | **[mM]** | **[mg/mL]** |
| **Furosemide^{a,b}** | 2.0 | 25 | 2 | (17 ± 4)·10⁻³ | (5.5 ± 1.2)·10⁻³ |
| **Furosemide** | 2.0 | 35.5 | 2 | (22 ± 2)·10⁻³ | (7.3 ± 0.8)·10⁻³ |
| **7a** | 6.8 | 35.5 | 1000 | 43.6 ± 1.4 | 14.5 ± 0.5 |
| **7b** | 6.8 | 35.5 | 1000 | 17.2 ± 0.6 | 6.1 ± 0.2 |
| **7c** | 6.8 | 35.5 | 500 | 13.6 ± 0.7 | 4.5 ± 0.2 |
| **19c** | 6.8 | 35.5 | 500 | 32.6 ± 0.8 | 8.9 ± 0.2 |

| | | | | | |
|---|---|---|---|---|---|
| ^{a} Three independent shake flask experiments performed in parallel, reported values are the mean of 9 concentration measurements. ^{b} cf. E. Baka et al. Journal of Pharmaceutical and Biomedical Analysis 2008, 46, 335: (18.7 ± 1.2)·10⁻³ mg/mL. | | | | | |

35.5°C is the mean temperature of the anterior part of the oral cavity (cf. Moore et al. European J. Orthod. 1999, 21, 249). pH 6.8 was chosen in accordance with the reported mean oral mucosal pH of various parts of the oral cavity (pH 6.78 ± 0.04, cf. Aframian et al. Oral Dis. 2006, 12, 420). The salivary pH is maintained by three buffer systems (bicarbonate, phosphate, and protein), and saliva's ionic strength is up to 70 mM (cf. Macakova et al. Tribology International 2011, 44, 956). Hence, the conditions chosen for the solubility assay closely resemble the conditions present during sublingual or buccal administration. Assuming 1.1 mL as saliva volume in the oral cavity (cf. Llena-Puy, Med. Oral. Patol. Oral Cir. Bucal. 2006, 11(5), E449), the mass of **7a** that is soluble in this volume is stoichiometrically equivalent to a psilocybin dose of 13.6 mg which is in the range of clinically studied oral psilocybin doses (cf. Johnson et al., Neurotherapeutics 2017, 14, 734). Considering the treatment setting, it may also be preferable to use multiple, smaller, fast-onset formulation doses in order to titrate the total dosage according to the subjective effects on the patient.

## Claims

1. A prodrug having the formula (1) or salts thereof, 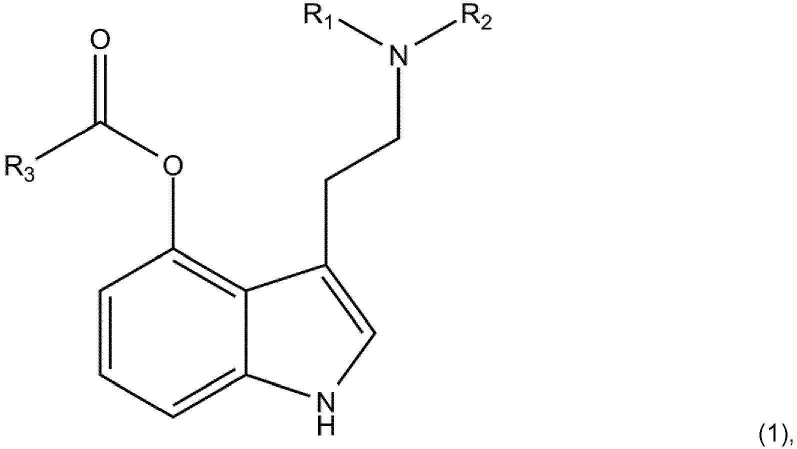
wherein R₃ is selected from the group consisting of cyclopropyl, isopropyl, *n*-propyl, isobutyl, *tert*-butyl, neopentyl, phenyl, and 2-furyl residues; and,
provided that R₃ is selected from the group consisting of cyclopropyl, isopropyl, and 2-furyl residues, R₁ and R₂ are selected from the group consisting of methyl, ethyl, *n-*propyl, isopropyl, *n*-butyl, *tert*-butyl residues and combinations thereof;
and, provided that R₃ is selected from the group consisting of *n*-propyl, isobutyl, *tert-*butyl, neopentyl, and phenyl residues, R₁ and R₂ are not the same and are selected from the group consisting of methyl, ethyl, *n*-propyl, isopropyl, *n*-butyl, and *tert*-butyl residues; and
wherein, in the salts of formula (1), the product of said formula (1) is protonated.

2. The prodrug according to claim 1, wherein an anion of the salts of formula (1) is selected from the group consisting of acetate, propionate, butyrate, malonate, maleate, succinate, fumarate, levulinate, and mesylate, particularly fumarate.

3. The prodrug according to claim 1 or 2, wherein R₃ is selected from the group consisting of cyclopropyl, isopropyl, and 2-furyl residues and R₁ and R₂ are selected from methyl and ethyl residues;
or wherein R₃ is phenyl, R₁ is a methyl residue, and R₂ is an ethyl residue.

4. The prodrug according to any one of claims 1 to 3, wherein R₃ is selected from cyclopropyl and isopropyl residues, R₁ is a methyl residue, and R₂ is an ethyl residue.

5. The prodrug according to any one of claims 1 to 4, having a passive membrane permeability Pₑ of at least 6 × 10⁻⁶ cm/s, measured as described in the description.

6. The prodrug according to any one of claims 1 to 5, having a half-life period of at least 15 min in human saliva at 37 °C, measured as described in the description.

7. A prodrug for use as a medicament, the prodrug having the formula (1) or salts thereof, 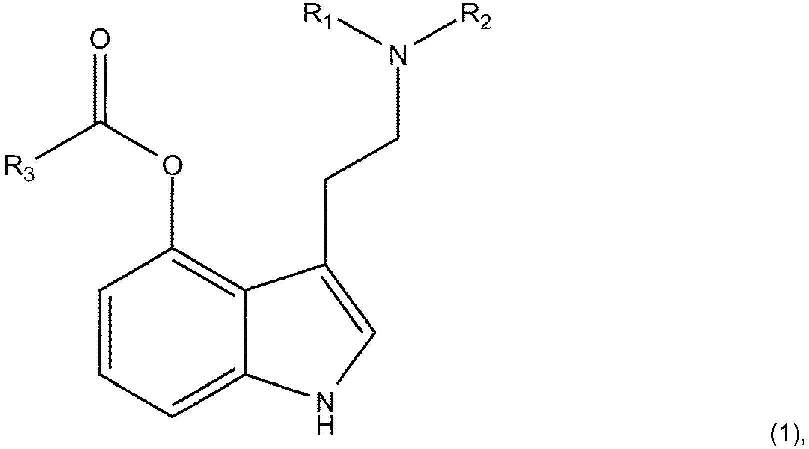
wherein R₃ is selected from the group consisting of cyclopropyl, isopropyl, *n*-propyl, isobutyl, *tert*-butyl, neopentyl, phenyl, and 2-furyl residues; and,
provided that R₃ is selected from the group consisting of cyclopropyl, isopropyl, and 2-furyl residues, R₁ and R₂ are selected from the group consisting of methyl, ethyl, *n-*propyl, isopropyl, *n*-butyl, *tert*-butyl residues and combinations thereof;
and, provided that R₃ is selected from the group consisting of *n*-propyl, isobutyl, *tert-*butyl, neopentyl, and phenyl residues, R₁ and R₂ are not the same and are selected from the group consisting of methyl, ethyl, *n*-propyl, isopropyl, *n*-butyl, and *tert*-butyl residues; and
wherein, in the salts of formula (1), the product of said formula (1) is protonated.

8. A prodrug for use in the treatment of psychiatric disorders, the prodrug having the formula (1) or salts thereof, 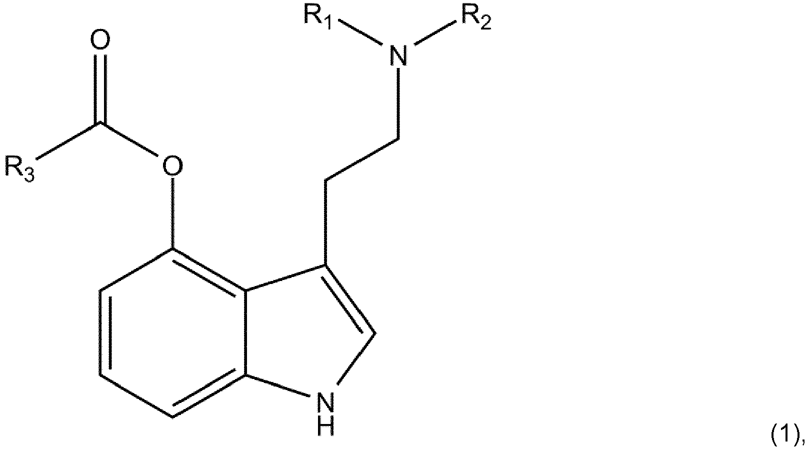
wherein R₃ is selected from the group consisting of cyclopropyl, isopropyl, *n*-propyl, isobutyl, *tert*-butyl, neopentyl, phenyl, and 2-furyl residues; and,
provided that R₃ is selected from the group consisting of cyclopropyl, isopropyl, and 2-furyl residues, R₁ and R₂ are selected from the group consisting of methyl, ethyl, *n-*propyl, isopropyl, *n*-butyl, *tert*-butyl residues and combinations thereof;
and, provided that R₃ is selected from the group consisting of *n*-propyl, isobutyl, *tert-*butyl, neopentyl, and phenyl residues, R₁ and R₂ are not the same and are selected from the group consisting of methyl, ethyl, *n*-propyl, isopropyl, *n*-butyl, and *tert*-butyl residues; and
wherein, in the salts of formula (1), the product of said formula (1) is protonated.

9. The prodrug for use in the treatment of psychiatric and/or neurological disorders according to claim 8, wherein the psychiatric and/or neurological disorder is one of depression, post-traumatic stress disorder, substance use disorder, anorexia nervosa, and neurodegenerative diseases, particularly Alzheimer's disease.

10. The prodrug for use in the treatment of psychiatric disorders according to claim 8 or 9, wherein the prodrug administration is buccal, sublingual, inhalatory, intranasal, and/or transdermal.

11. The prodrug for use in the treatment of psychiatric disorders according to any one of claims 8 to 10, wherein the treatment includes psychedelic therapy.

12. A prodrug having the formula (2) or salts thereof, 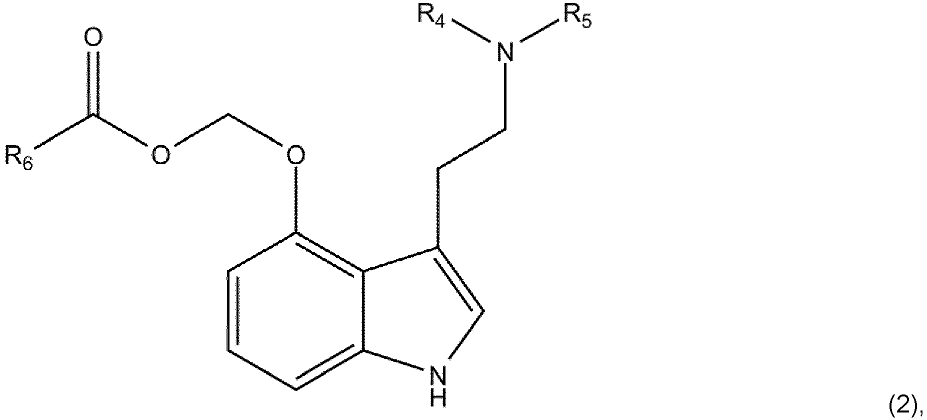
wherein R₆ is selected from the group consisting of cyclopropyl, isopropyl, *n*-propyl, isobutyl, *tert*-butyl, neopentyl, phenyl, and 2-furyl residues; and,
provided that R₆ is selected from the group consisting of isopropyl and 2-furyl residues, R₄ and R₅ are selected from the group consisting of methyl, ethyl, *n*-propyl, isopropyl, *n*-butyl, *tert*-butyl residues and combinations thereof;
and, provided that R₆ is selected from the group consisting of *n*-propyl, cyclopropyl, isobutyl, *tert*-butyl, neopentyl, and phenyl residues, R₄ and R₅ are not the same and are selected from the group consisting of methyl, ethyl, *n*-propyl, isopropyl, *n*-butyl, and *tert*-butyl residues; and
wherein, in the salts of formula (2), the product of said formula (2) is protonated.

13. A prodrug for use as a medicament, the prodrug having the formula (2) or salts thereof, 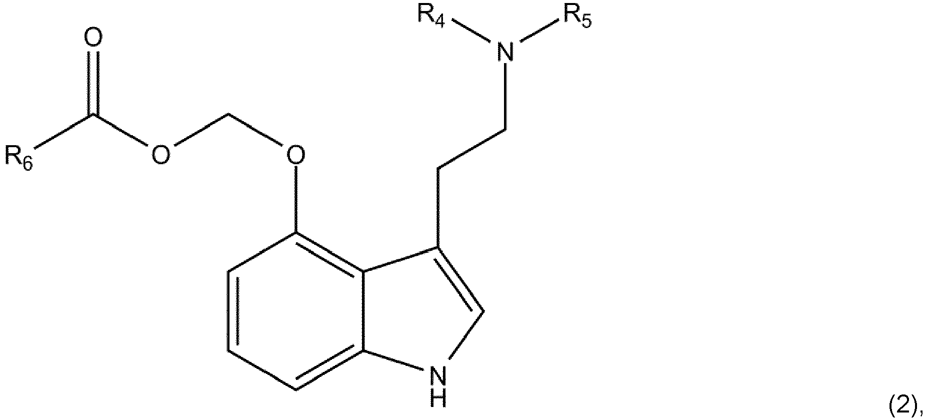
wherein R₆ is selected from the group consisting of cyclopropyl, isopropyl, *n*-propyl, isobutyl, *tert*-butyl, neopentyl, phenyl, and 2-furyl residues; and,
provided that R₆ is selected from the group consisting of isopropyl and 2-furyl residues, R₄ and R₅ are selected from the group consisting of methyl, ethyl, *n*-propyl, isopropyl, *n*-butyl, *tert*-butyl residues and combinations thereof;
and, provided that R₆ is selected from the group consisting of *n*-propyl, cyclopropyl, isobutyl, *tert*-butyl, neopentyl, and phenyl residues, R₄ and R₅ are not the same and are selected from the group consisting of methyl, ethyl, *n*-propyl, isopropyl, *n*-butyl, and *tert*-butyl residues; and
wherein, in the salts of formula (2), the product of said formula (2) is protonated.

14. A prodrug for use in the treatment of psychiatric disorders, the prodrug having the formula (2) or salts thereof, 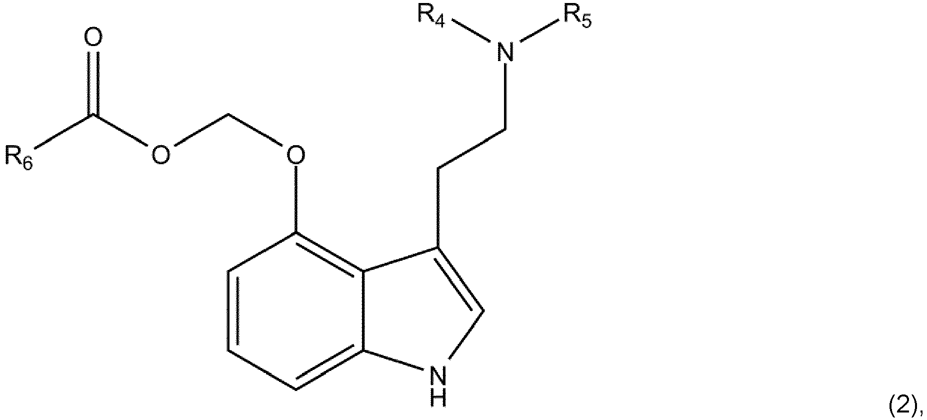
wherein R₆ is selected from the group consisting of cyclopropyl, isopropyl, *n*-propyl, isobutyl, *tert*-butyl, neopentyl, phenyl, and 2-furyl residues; and,
provided that R₆ is selected from the group consisting of isopropyl and 2-furyl residues, R₄ and R₅ are selected from the group consisting of methyl, ethyl, *n*-propyl, isopropyl, *n*-butyl, *tert*-butyl residues and combinations thereof;
and, provided that R₆ is selected from the group consisting of *n*-propyl, cyclopropyl, isobutyl, *tert*-butyl, neopentyl, and phenyl residues, R₄ and R₅ are not the same and are selected from the group consisting of methyl, ethyl, *n*-propyl, isopropyl, *n*-butyl, and *tert*-butyl residues; and
wherein, in the salts of formula (2), the product of said formula (2) is protonated.
